# FASCICULE DE BREVET EUROPEEN

(11) **EP 4 149 376 B1**
(45) Date de publication et mention de la délivrance du brevet: **29.01.2025**
(21) Numéro de dépôt: 21724311.2
(22) Date de dépôt: 11.05.2021
(51) Int. Cl.: A61B 17/70

(54) **DISPOSITIF IMPLANTABLE, NOTAMMENT DE TYPE ESPACEUR INTERVERTÉBRAL**
IMPLANTIERBARE VORRICHTUNG, INSBESONDERE VOM TYP EINES ZWISCHENWIRBEL-SPACERS
IMPLANTABLE DEVICE, IN PARTICULAR OF THE INTERVERTEBRAL SPACER TYPE

(30) Priorité: 14.05.2020 FR 2004780; 02.02.2021 FR 2100980
(43) Date de publication de la demande: 22.03.2023
(73) Titulaire: Cousin Biotech, 59117 Wervicq Sud (FR)
(72) Inventeur: HOFFMANN, Eléonore, 59118 WAMBRECHIES (FR); BECQUART, Lucie, 59800 LILLE (FR); CAILLIBOTTE, Michel, 59800 LILLE (FR)
(74) Mandataire: Cabinet Beau de Loménie
(86) Numéro de dépôt international: PCT/EP2021/062514
(87) Numéro de publication internationale: WO 2021/228876

(56) Documents cités:
- FR-A1- 2 844 179
- US-A1- 2006 282 075
- US-A1- 2008 183 209
- US-A1- 2010 131 009
- US-A1- 2013 023 990
- US-A9- 2018 368 890

## Description

### Domaine Technique

La présente invention a pour objet un dispositif implantable, notamment de type espaceur intervertébral, pour le traitement d'au moins un niveau du rachis.

### Technique antérieure

Ces dispositifs implantables, notamment de type espaceurs intervertébraux, ont pour fonction de répartir les charges et surcharges entre deux vertèbres adjacentes (désignés dans le présent texte par vertèbres sus et sous-jacentes), par exemple créées par la dégénérescence discale (notamment dans le cas d'une hernie discale).

Les vertèbres de la colonne vertébrale humaine sont disposées dans une colonne avec une vertèbre au-dessus de la suivante. Le disque intervertébrale se trouve entre les vertèbres sus et sous-jacentes pour transmettre les efforts entre les vertèbres et pour amortir ces efforts.

Les disques permettent également à la colonne vertébrale de fléchir (lors des mouvements de flexion et d'extension du sujet) et de se tordre (lors des mouvements de flexion et d'extension du sujet). Avec le temps, les disques intervertébraux se dégradent ou autrement dit se dégénèrent. Cette dégénérescence entraîne une perte de liquide dans les disques et les rend moins flexibles. De même, les disques deviennent plus fins permettant aux vertèbres de se rapprocher. La dégénérescence peut également entraîner des déchirures ou des fissures dans la couche externe, ou l'anneau, du disque. Le disque peut commencer à gonfler à l'extérieur du disque.

Dans toutes ces conditions, les espaces parcourus par la moelle épinière et les racines nerveuses rachidiennes peuvent se rétrécir, entraînant une pression sur le tissu nerveux qui peut causer de la douleur, un engourdissement, une faiblesse, ou même une paralysie dans diverses parties du corps. Enfin, les articulations facettaires entre les vertèbres adjacentes peuvent dégénérer et provoquer une douleur localisée et/ou rayonnante.

Tous les symptômes et troubles ci-dessus sont considérés individuellement ou en combinaison comme une maladie du rachis.

Afin de les traiter, les chirurgiens essayent de rétablir l'espacement normal entre les vertèbres adjacentes. Cela peut être suffisant pour soulager la pression du tissu nerveux affecté. Cependant, il est souvent nécessaire d'enlever également chirurgicalement le matériau du disque, l'os ou d'autres tissus qui empiètent sur le tissu nerveux et/ou de débrider les articulations des facettes. Le plus souvent, la restauration de l'espacement intervertébral est accomplie en insérant une entretoise solide faite d'os, de métal ou de plastique dans l'espace discal entre les vertèbres adjacentes.

Les techniques de mise en place des espaceurs intervertébraux sont invasives, généralement en chirurgie ouverte, car elles nécessitent le placement du matériel prothétique en profondeur dans le site chirurgical adjacent à la colonne vertébral. Le rétablissement d'une telle intervention peut nécessiter plusieurs jours d'hospitalisation et une réadaptation lente et prolongée à des niveaux d'activité normale.

Il existe ainsi un besoin pour des espaceurs intervertébraux adaptés pour pouvoir être insérés via des techniques chirurgicales qui sont le moins invasives possibles. Un exemple d'une telle technique chirurgicale est celle développée par la société Tsunami mettant en jeu une implantation par la voie percutanée latérale, mais sans trocart. Elle consiste par exemple à réaliser au moins une incision, puis à passer un guide fil à travers cette incision pour guider le placement du dispositif implantable sur le niveau de la colonne vertébrale à traiter à travers les tissus. La technique de fluoroscopie peut être utilisée en parallèle afin d'assister le chirurgien dans le placement du dispositif implantable. Néanmoins, dans cette technique, le dispositif implantable est forcé à travers les tissus le long du guide fil, et peut ainsi les endommager.

Une autre technique d'intervention sur le rachis est une technique dite mini-invasive (MISS pour Minimally Invasive Spine Surgery) mettant en jeu au moins deux trocarts ou conduits (tube(s)). Un premier conduit reçoit une caméra et un second conduit permet le passage du dispositif implantable et du ou des outil(s) chirurgical(aux). Cette technique a pour avantage que le passage jusqu'à la zone du rachis à traiter est encadré par l'un des conduits de sorte que le dispositif implantable n'abime pas de tissus lors de sa disposition sur le site du rachis à traiter.

Cette technique MISS n'est pas encore utilisée pour les espaceurs intervertébraux, car ces derniers sont volumineux et dans des matériaux rigides afin de maintenir un écartement intervertébral minimum une fois implanté.

Certains espaceurs vertébraux inter-épineux sont agencés pour être implantés par voie percutanée à l'aide d'un guide fil. Un tel espaceur, par exemple celui commercialisé sous le nom de Superion par la société Vertiflex, comprend des parties montées pivotantes sur un support afin de présenter une configuration compacte dans la position d'insertion et une position déployée dans la position d'implantation. Les parties rigides sont pivotées par le chirurgien de la position d'introduction à la position d'implantation, et maintenues dans cette dernière position à l'aide d'un mécanisme de serrage. La stabilité sur le niveau du rachis instrumenté est également obtenue par le serrage des parties pivotantes sur les épineuses.

Il existe ainsi un risque que ces parties pivotantes cassent sous l'effet des charges et des surcharges. Par ailleurs, ce dispositif implantable ne peut être mis en oeuvre à l'aide d'une des techniques MISS consistant à placer un seul trocart latéralement par rapport au plan sagittal passant par le ligament supra épineux. En effet, il est recommandé, lorsque que l'on souhaite placer un dispositif implantable par une voie postérieure, que le trocart ne soit pas dans le même plan par rapport à celui passant par ligament supraépineux afin de ne pas endommager celui-ci. Ce qui sous-entend que le dispositif doit sortir du trocart pour se positionner sur le site opératoire inter-lamaire ou inter-épineux en négociant un virage. Or, le dispositif SUPERION destiné à être implanté dans une position inter-épineuse ne pourrait pas être positionné selon cette technique, car il est trop encombrant et rigide pour pouvoir passer facilement ce virage. De ce fait, ce dispositif SUPERION est placé dans un trocart qui traverse le ligament supraépineux, et donc endommage le ligament supraépineux de façon irréversible.

### Exposé de l'invention

La présente invention est définie dans la revendication 1, tandis que les modes de réalisation préférés sont décrits dans les revendications dépendantes. Les méthodes associées sont également décrites ici pour faciliter la compréhension de l'invention.

La présente invention a ainsi pour objet un dispositif implantable, en particulier pour le maintien d'un écart intervertébral entre des vertèbres sus et sous-jacentes qui peut être implanté via une technique de chirurgie peu invasive, en percutanée ou par la voie mini-invasive (MISS) via des trocarts.

La présente invention a pour objet un dispositif implantable permettant de maintenir un écartement intervertébral, en particulier interlamaire, sur le niveau de la colonne vertébral traité, tout en apportant une stabilité dynamique, c'est-à-dire permettant une certaine mobilité entre les vertèbres sus et sous-jacentes traitées.

Le dispositif implantable selon l'invention peut être utilisé, à titre non limitatif, pour traiter des pathologies du type hernies du disque, sténose du canal rachidien, et/ou une dégénérescence du disque.

La présente invention a pour objet, selon un premier aspect, un dispositif implantable, notamment pour maintenir un écartement intervertébral entre des vertèbres sus et sous-jacentes, comprenant :
- une cale intervertébrale comprenant une zone supérieure d'appui, en particulier sur au moins une portion de la lame de la vertèbre sus-jacente, et une zone inférieure d'appui, en particulier sur au moins une portion de la lame de la vertèbre sous-jacente, distantes d'une distance Da minimum pour le maintien d'un écart intervertébral, et
- au moins un premier ressort latéral ayant une position A par rapport à la cale intervertébrale dans une position d'introduction du dispositif implantable, et une position B par rapport à la cale intervertébrale dans une position d'implantation du dispositif implantable, les positions A et B étant différentes. Le premier ressort latéral est configuré :
- pour passer de la position A à la position B librement, et
- former un organe de retenue agencé par rapport à la cale intervertébrale pour bloquer la migration de la cale intervertébrale vers le canal rachidien au moins dans la position B. Avantageusement, le dispositif implantable comprend une partie de sa structure formée d'un premier ressort latéral, et éventuellement d'un second ressort latéral (similaire au premier ressort latéral), permettant de réduire l'encombrement du dispositif implantable par déformation de ce dernier de manière réversible pour le placer dans une position d'introduction. Du fait de la structure inhérente et propre à un ressort, en particulier de ses propriétés élastiques, le premier ressort latéral récupère sa forme d'implantation automatiquement, rapidement ou de manière progressive, une fois libéré sur le site d'implantation, sans déformation irréversible et/ou serrage exercé à l'aide d'au moins un moyen de serrage et d'un ancillaire de manipulation dudit dispositif implantable permettant son passage de la position d'introduction à la position d'implantation.

De préférence, le premier ressort latéral comprend une première région qui se rapproche ou s'éloigne de la cale intervertébrale lors de son placement dans la position A, correspondant à la position d'introduction du dispositif implantable.

De préférence, le second ressort latéral comprend une seconde région qui se rapproche ou s'éloigne de la cale intervertébrale lors de son placement dans la position A, correspondant à la position d'introduction du dispositif implantable.

On comprend par position d'introduction, la position dans laquelle le dispositif implantable a un encombrement réduit dans au moins une direction par rapport à sa position d'implantation, et/ou la position dans laquelle le dispositif implantable est contraint pour se déformer, notamment élastiquement, facilitant ainsi son introduction dans un conduit de diamètre interne déterminé, notamment un trocart. Le dit tube peut avoir un diamètre interne supérieur ou égal à 5 mm et inférieur ou égal à 50 mm, par exemple de l'ordre de 12 mm.

On comprend par position d'implantation, la position adoptée par le dispositif implantable sur le niveau du rachis traité et/ou la position du dispositif implantable libre de toute contrainte visant à le déformer pour son maintien dans la position d'introduction, en particulier à une température supérieure ou égale à As (Température Austenite Start), avec par exemple une As de l'ordre de 15°C, notamment pour un alliage Titane Nickel.

Dans un mode de réalisation, le premier ressort latéral (et/ou le second ressort latéral) est(sont) dans un plan frontal F1 (ou dans des plans frontaux F1 et F2 respectifs), notamment sensiblement parallèle(s) au plan frontal F de la cale intervertébrale, dans la position A et la position B. Par exemple, le premier ressort latéral et/ou le second ressort latéral a/(ont chacun) une hauteur qui est augmentée ou diminuée dans ledit plan frontal F1 et/ou dans ledit plan frontal F2.

De préférence, les plans F1 et F2 sont sensiblement parallèles, en particulier confondus.

Dans un autre mode de réalisation, le premier ressort latéral est dans un plan sagittal S1 et/ou le second ressort latéral est dans un plan sagittal S2, notamment sensiblement parallèle(s) au plan sagittal S de la cale intervertébrale, dans la position A ; et le premier ressort latéral est dans un plan frontal F1 et/ou le second ressort latéral est dans un plan frontal F2, dans la position B, notamment sensiblement parallèle(s) au plan frontal F de la cale intervertébrale, les plans S1 et/ou S2 étant sécants avec le ou les plans F1 et/ou F2, en particulier sensiblement perpendiculaires. Par exemple, le premier ressort latéral et/ou le second ressort latéral est/sont rabattu(s) contre la cale intervertébrale dans la position A.

Le premier ressort latéral, et éventuellement le second ressort latéral décrit ci-après, est/sont placé(s) dans la position A, correspondant à la position d'introduction du dispositif implantable, en appliquant une contrainte mécanique consistant à comprimer le premier (ou second) ressort latéral, par exemple à l'aide d'un ancillaire et/ou en disposant le dispositif implantable dans un tube, cette contrainte permet de rapprocher ou d'écarter deux régions distinctes du premier ressort latéral et/ou du second ressort latéral, et donc de le, ou les, déformer élastiquement.

De préférence, le premier ressort latéral et/ou le second ressort latéral, a/ont plusieurs positions A dans lesquelles ils sont déformés. Cependant, une position A de déformation est privilégiée pour le fonctionnement du ou des premier et/ou second ressort(s) latéral(aux) selon la construction du dispositif implantable.

De préférence, le premier ressort latéral et/ou le second ressort latéral comprend/ comprennent au moins un matériau comprenant au moins un métal ou un alliage métallique, implantable et élastique, par exemple un acier inoxydable élastique et/ou un matériau à mémoire de forme.

On comprend par au moins un métal élastique, tout métal capable de subir des déformations de manière réversible, en particulier à une température comprise entre 0°C et 40°C.

Le premier ressort latéral, et éventuellement le second ressort latéral, est/sont placé(s) dans la position B, correspondant à la position d'implantation du dispositif implantable, en ôtant la contrainte mécanique de déformation appliquée dans la position A, et/ou par apport thermique activant le retour à une forme dite d'éducation correspondant à la position B du premier et/ou du second ressort(s) latéral(aux).

De préférence, le premier ressort latéral (et éventuellement le second ressort latéral) se positionne(nt) dans la position B librement, sous l'effet de son élasticité intrinsèque et/ou sous l'effet d'une activation thermique, en particulier sans assistance extérieure audit dispositif, par exemple du type dispositif de serrage maintenant le dispositif implantable dans sa position d'implantation.

En particulier, le premier ressort latéral (et éventuellement le second ressort latéral) se positionne(nt) dans la position B du fait de ses propriétés intrinsèques : c'est-à-dire de sa construction, notamment comprenant une ou plusieurs zone(s) de ressort mécanique, et/ou, du ou des matériau(x) qu'il(s) comprend/comprennent, par exemple au moins un matériau à mémoire de forme et/ou au moins un métal élastique.

L'insertion du dispositif implantable selon l'invention sur le site du rachis à traiter permet avantageusement de ne pas endommager le ligament supraépineux. En effet, le dispositif implantable peut être inséré et acheminé dans sa position d'introduction dans un trocart, disposé à distance, à droite ou à gauche du ligament supraépineux, et comprenant éventuellement un coude à son extrémité distale.

On comprend par « librement » qu'aucune contrainte mécanique externe, notamment permanente (par l'intermédiaire d'un dispositif ou d'un ancillaire de serrage et/ou de verrouillage) n'est appliquée au premier ressort latéral (et/ou au second ressort latéral) pour permettre sa disposition dans la position B à partir de sa position A.

Dans un mode de réalisation préféré, le dispositif implantable comprend un second ressort latéral ayant la position A par rapport à la cale intervertébrale dans une position d'introduction du dispositif implantable, et la position B par rapport à la cale intervertébrale dans une position d'implantation du dispositif implantable, les positions A et B étant différentes. Le second ressort latéral est configuré :
- pour passer de la position A à la position B librement, et
- former un organe de retenue agencé par rapport à la cale intervertébrale pour bloquer la migration de la cale intervertébrale vers le canal rachidien au moins dans la position B.

De préférence, tout ce qui est décrit dans le présent texte ((sous)variantes de réalisation, définitions, propriétés, modes de réalisation,...) en référence au premier ressort latéral, s'applique intégralement au second ressort latéral.

Dans un mode de réalisation, le premier ressort latéral et/ou le second ressort latéral est/sont déformable(s), notamment élastiquement, de manière réversible, en particulier à une température supérieure ou égale 0°C et inférieure ou égale à 37°C.

On comprend par déformable de manière réversible que le premier ressort latéral et/ou le second ressort latéral peut/peuvent être déformé(s) pour passer de la position A à la position d'implantation B, et inversement.

On comprend par plan de coupe sagittal dans le présent texte, le plan de coupe sagittal S de la cale intervertébrale selon l'invention, en particulier correspondant sensiblement au plan de coupe sagittal du corps humain passant sensiblement par le plan de coupe sagittal S de la cale intervertébrale du dispositif implantable dans sa position d'implantation.

On comprend par plan de coupe frontal F dans le présent texte, le plan de coupe frontal F de la cale intervertébrale selon l'invention, en particulier correspondant sensiblement au plan de coupe frontal du corps humain passant sensiblement par le plan de coupe frontal F de la cale intervertébrale du dispositif implantable dans sa position d'implantation.

On comprend par plan de coupe transverse T dans le présent texte, le plan de coupe transverse T de la cale intervertébrale selon l'invention, en particulier correspondant sensiblement au plan de coupe transverse du corps humain passant sensiblement par le plan de coupe transverse T de la cale intervertébrale du dispositif implantable dans sa position d'implantation.

La cale intervertébrale selon l'invention est configurée pour maintenir un écartement intervertébral entre les vertèbres sus et sous-jacentes, en particulier de manière à stabiliser dynamiquement le niveau du rachis instrumenté.

La cale intervertébrale est de préférence dimensionnée pour combler la zone d'implantation, c'est-à-dire la zone interlamaire traitée pour être plus proche du centre de rotation des vertèbres sus et sous-jacentes, et se positionner sur la zone la plus solide des vertèbres, à savoir les lames.

De préférence, la cale intervertébrale est configurée pour pouvoir se comprimer, légèrement, notamment dans une direction s'étendant de la zone supérieure d'appui vers la zone inférieure d'appui, en utilisation, pour conserver les mouvements du segment vertébral traité.

Les expressions configuré pour, adapté pour et apte à, sont équivalentes dans le présent texte.

Dans des modes de réalisation, la cale intervertébrale comprend des première et seconde zones latérales, notamment disposées de part et d'autre d'un plan de coupe sagittal S de la cale intervertébrale. La cale intervertébrale comprend de préférence des zones avant et arrière disposées de part et d'autre d'un plan de coupe frontal F de la cale intervertébrale.

De préférence, les premier et second ressorts latéraux sont disposés de part et d'autre du plan de coupe sagittal (S) de la cale intervertébrale.

De préférence, les premier et second ressorts latéraux s'étendent respectivement des première et seconde zones latérales de la cale intervertébrale.

Dans un mode de réalisation, la cale intervertébrale comprend au moins un matériau polymère, en particulier élastomérique, notamment (sur)moulé, et/ou au moins un textile.

Le matériau polymère peut être choisi parmi les silicones et les polyuréthanes, ou une combinaison de ces derniers.

Ledit textile peut être un tricot, un tissu, un non-tissé, une tresse, un câble ou une combinaison de ces derniers.

De préférence, le textile comprend un ou plusieurs fil(s) monofilamentaire(s) et/ou un plusieurs fil(s) multifilamentaire(s) et/ou un ou plusieurs fil(s) filés de fibres.

Le ou les fil(s) défini(s) dans le présent texte peut/peuvent comprendre (être constitué(s)) d'un ou plusieurs matériau(x) polymère(s) biocompatible(s) résorbable(s), partiellement ou totalement, ou non résorbable(s).

Le ou lesdits matériau(x) peut/peuvent être choisi(s) parmi : les polyoléfines, tels que le polypropylène, le polyéthylène ; les polyesters, tels que le polyéthylène téréphtalate d'éthylène ou de butylène ; les polyuréthanes ; les polymères résorbables, notamment comprenant de l'acide polylactique (PLLA, PLDA,...).

Dans une variante, ledit au moins premier ressort latéral a une hauteur H1 dans la position A et une hauteur H2 dans la position B, H2 étant différente de H1.

Dans un premier mode de réalisation, H1 est inférieure à H2.

Dans un second mode de réalisation, H1 est supérieure à H2.

De préférence, le dispositif implantable comprend un second ressort latéral ayant une hauteur H11 dans la position A, et une hauteur H22 dans la position B, H22 étant différente de H11.

Dans un premier mode de réalisation, H11 est inférieure à H22.

Dans un second mode de réalisation, H11 est supérieure à H22.

Le premier mode de réalisation et le second mode de réalisation correspondent à deux positions A différentes du/des premier et/ou second ressort(s) latéral(aux).

Dans une variante de réalisation, le premier ressort latéral comprend au moins un matériau choisi parmi les matériaux à mémoire de forme.

Les matériaux à mémoire de forme sont de préférence des matériaux à mémoire de forme métalliques.

Ledit au moins un matériau à mémoire de forme peut être choisi parmi : un alliage comprenant au moins du titane, et éventuellement du nickel ; en particulier le titane et le nickel sont dans des proportions massiques sensiblement égales. Ledit alliage peut être le nitinol.

De préférence, le second ressort latéral comprend au moins un matériau choisi parmi les matériaux à mémoire de forme, notamment métalliques.

Le premier ressort latéral et/ou le second ressort latéral a/ont été éduqué(s) (en particulier par application d'un traitement thermique lors de leur fabrication) selon une forme déterminée, en particulier à la forme B selon l'invention, en sorte que le premier ressort latéral et/ou le second ressort latéral recouvre(nt) ladite forme B par activation thermique de la forme B mémorisée.

Ledit matériau à mémoire de forme est de préférence déterminé, en particulier sa composition, selon les températures austénite start (As) et austénite finish (Af) envisagées. La température As définit la température à partir de laquelle le matériau à mémoire de forme commence à bouger vers sa forme d'éducation, et Af la température à laquelle le matériau à mémoire de forme a retrouvé sa forme d'éducation.

A titre d'exemple, As peut être de l'ordre de 15°C pour le matériau à mémoire de forme sélectionné pour le premier et/ou le second ressort(s) latéral(aux). Ainsi, à une température inférieure à environ 15°C, le matériau à mémoire de forme est dans un état plastique, et à une température supérieure à environ 15°C, le matériau est dans un état superélastique. As peut être de l'ordre de 35°C-37°C par exemple afin que le matériau à mémoire de forme soit dans un état plastique à température ambiante, et superélastique à la température corporelle, une fois implanté.

Il est possible également de stocker le dispositif implantable dans sa position d'implantation, par exemple disposé dans un tube creux, à température ambiante ou dans un dispositif réfrigérant afin que le ou les ressort(s) soit/soient dans un état plastique, et donc aisément déformables.

L'homme du métier connaissant les matériaux à mémoire de forme sait adapter la composition de l'alliage métallique afin d'ajuster les températures As et Af selon les conditions d'implantation désirées.

Dans une variante de réalisation, le premier ressort latéral comprend des premières portions supérieure et inférieure distantes de D1 dans la position A, et distantes de D2 dans la position B, D2 étant différente de D1, et le dispositif implantable est positionné dans la position d'introduction par rapprochement ou écartement des premières portions supérieure et inférieure du premier ressort latéral.

Lorsque les premières portions supérieure et inférieure sont rapprochées l'une de l'autre, D2 est supérieure à D1, et lorsqu'elles sont écartées, D2 est inférieure à D1.

Dans des modes de réalisation, le second ressort latéral comprend des secondes portions supérieure et inférieure distantes de D11 dans la position A, et distantes de D22 dans la position B, D22 étant différente, par exemple supérieure ou inférieure à D11, et le dispositif implantable est positionné dans la position d'introduction par rapprochement ou écartement des secondes portions supérieure et inférieure du second ressort latéral.

Dans des modes de réalisation, la distance D1 correspond sensiblement à la hauteur H1, et la distance D2 correspondant sensiblement à la hauteur H2, et/ou la distance D11 correspond sensiblement à la hauteur H11, et la distance D22 correspondant sensiblement à la hauteur H22.

De préférence, les premières portions supérieure et inférieure du premier ressort latéral se projettent de part et d'autre d'un plan de coupe transverse T de la cale intervertébrale.

De préférence, les secondes portions supérieure et inférieure du second ressort latéral se projettent de part et d'autre d'un plan de coupe transverse T de la cale intervertébrale.

Dans une variante de réalisation, la cale intervertébrale a une hauteur maximum Ha, et Ha est inférieure à H2, et/ou à H22, dans la position d'implantation.

Avantageusement, le premier ressort latéral, et éventuellement le second ressort latéral, se projette(nt) au-dessus de la zone supérieure d'appui de la cale intervertébrale et/ou au-dessous de la zone inférieure d'appui de la cale intervertébrale.

Cette disposition permet avantageusement pour le premier ressort latéral, et éventuellement le second ressort latéral, de bloquer la migration de la cale intervertébrale vers le canal rachidien.

Dans une variante de réalisation, le premier ressort latéral s'étend d'un côté d'un plan de coupe sagittale (S) de la cale intervertébrale.

Dans des modes de réalisation, le second ressort latéral s'étend d'un côté d'un plan de coupe sagittale (S) de la cale intervertébrale, notamment de la seconde zone latérale de la cale intervertébrale.

Dans une variante de réalisation, le premier ressort latéral comprend une première armature comprenant un ou plusieurs élément(s) longiligne(s) au moins en partie métallique(s), tel un ruban ou un fil.

Dans des modes de réalisation, le second ressort latéral comprend une seconde armature comprenant un ou plusieurs élément(s) longiligne(s) au moins en partie métallique(s), tel un ruban ou un fil.

La section du ou des élément(s) longiligne(s) métallique(s) peut être sensiblement circulaire ou ovale ou parallélépipédique, notamment rectangulaire.

Le ou les élément(s) longiligne(s) peut/peuvent comprendre un ou plusieurs matériau(x) métallique(s) à mémoire de forme et/ou un ou plusieurs matériau(x) métallique(s) à base d'acier inoxydable élastique.

De préférence, la première armature et/ou la seconde armature est/sont constituée(s) du ou des élément(s) longiligne(s) au moins en partie métallique(s) selon l'invention.

Dans des modes de réalisation, la première armature et/ou la seconde armature comprend/comprennent chacune une ou plusieurs zone(s) de pliage, notamment arrangées dans le ou les élément(s) longiligne(s) au moins en partie métallique(s) selon l'invention. En particulier, l'épaisseur d'une zone de pliage est inférieure à l'épaisseur moyenne de l'élément longiligne comprenant ladite zone de pliage.

La ou lesdites zone(s) de pliage forme(nt) de préférence une ou des lame(s) ressort.

Dans des modes de réalisation, la première armature et la seconde armature comprennent au moins un élément longiligne, au moins en partie métallique, en commun, ou, comprennent chacune un ou des élément(s) longiligne(s) au moins en partie métallique(s) distincts.

Dans un mode de réalisation, la cale intervertébrale est surmoulée sur : au moins une partie de la première armature et/ou sur au moins une partie de la seconde armature et/ou sur au moins une partie de l'armature principale (définie ci-après). Cette disposition permet de solidariser de manière fiable la cale intervertébrale à la, ou aux, première et/ou seconde armature(s), et/ou à l'armature principale.

Dans une variante de réalisation, la première armature comprend une première branche supérieure et une première branche inférieure qui divergent l'une de l'autre.

Dans des modes de réalisation, la seconde armature comprend une seconde branche supérieure et une seconde branche inférieure qui divergent l'une de l'autre.

De préférence, la première branche supérieure et/ou la première branche inférieure et/ou la seconde branche supérieure et/ou la seconde branche inférieure comprend/comprennent chacune au moins une portion en liaison avec la cale intervertébrale.

Dans des modes de réalisation, la première branche supérieure passe sensiblement par un axe L1, et la première branche inférieure passe sensiblement par un axe L2, L1 et L2 étant sécants et formant un angle α supérieur ou égal à 45°, en particulier supérieur ou égal à 90°.

Dans des modes de réalisation, la seconde branche supérieure passe sensiblement par un axe L3, et la seconde branche inférieure passe sensiblement par un axe L4, L3 et L4 étant sécants et formant un angle β supérieur ou égal à 45°, en particulier supérieur ou égal à 90°.

Dans des modes de réalisation, les premières branches supérieure et inférieure et/ou les secondes branches supérieure et inférieure comprennent (chacune) une extrémité libre.

Dans une variante de réalisation, la première armature comprend une première branche intermédiaire, continue ou discontinue, en liaison avec la première branche supérieure et avec la première branche inférieure.

Dans des modes de réalisation, la seconde armature comprend une seconde branche intermédiaire, continue ou discontinue, en liaison avec la seconde branche supérieure et avec la seconde branche inférieure.

On comprend par discontinue que la première branche intermédiaire, et/ou la seconde branche intermédiaire, est/sont interrompues.

Dans une sous-variante de réalisation, la première branche intermédiaire comprend des premières portions intermédiaires supérieure et inférieure convergent vers une première zone intermédiaire autour de laquelle lesdites premières portions intermédiaires supérieure et inférieure pivotent lors du passage de la position A vers la position B.

Dans des modes de réalisation, la seconde branche intermédiaire comprend des secondes portions intermédiaires supérieure et inférieure convergent vers une seconde zone intermédiaire autour de laquelle lesdites secondes portions intermédiaires supérieure et inférieure pivotent lors du passage de la position A vers la position B.

Les premières (ou secondes) portions intermédiaires supérieure et inférieure peuvent pivoter également autour de la première (ou seconde) zone intermédiaire pour passer de la position B vers la position A.

Dans des modes de réalisation, la première portion intermédiaire supérieure passe sensiblement par un axe A1, et la première portion intermédiaire inférieure passe sensiblement par un axe A2, A1 et A2 étant sécants et formant un angle supérieur ou égal à 45°, en particulier supérieur ou égal à 90°.

Dans des modes de réalisation, la seconde portion intermédiaire supérieure passe sensiblement par un axe A3, et la seconde portion intermédiaire inférieure passe sensiblement par un axe A4, A3 et A4 étant sécants et formant un angle supérieur ou égal à 45°, en particulier supérieur ou égal à 90°.

Dans une sous-variante de réalisation, les premières portions intermédiaires supérieure et inférieure comprennent chacune une extrémité libre.

Dans des modes de réalisation, les secondes portions intermédiaires supérieure et inférieure comprennent chacune une extrémité libre.

En particulier, la première zone intermédiaire de la première branche intermédiaire est une zone d'interruption de cette dernière.

En particulier, la seconde zone intermédiaire de la seconde branche intermédiaire est une zone d'interruption de cette dernière.

Dans une sous-variante, la première zone intermédiaire est une zone de pliage de la première branche intermédiaire.

Dans des modes de réalisation, la seconde zone intermédiaire est une zone de pliage de la seconde branche intermédiaire.

Dans une variante de réalisation, la première armature comprend au moins une portion d'élément longiligne faisant office de lame ressort, en particulier la première branche intermédiaire comprend au moins une lame ressort.

Dans des modes de réalisation, la seconde armature comprend au moins une portion d'élément longiligne faisant office de lame ressort, en particulier la seconde branche intermédiaire comprend au moins une lame ressort.

Dans une variante de réalisation, l'un au moins du ou desdits élément(s) longiligne(s) au moins en partie métallique comprend au moins une portion vrillée dans la position A ou B, et ladite portion vrillée est dévrillée lors de son changement de position entre les positions A et B. De préférence, le premier ressort latéral et/ou le second ressort latéral comprend/ comprennent chacun au moins une portion vrillée.

Par exemple, cette disposition peut être obtenue par passage du premier (ou second) ressort latéral de la position B vers la position A, le premier (ou second) ressort étant dans un plan frontale F1 (ou F2) dans la position B, et dans un plan sagittal S1 (ou S2) sensiblement sécant à F1 (ou F2), en particulier sensiblement perpendiculaire à F1 (ou F2), dans la position A. Le premier (ou second) ressort latéral est alors déformé au point qu'au moins une portion d'élément longiligne est vrillée. Lors du passage du premier (ou second) ressort latéral dans la position B, la portion vrillée est retordue en sens inverse, et donc dévrillée.

Cette variante définit une troisième position A pour le premier et/ou le second(s) ressort(s) latéral(aux).

De préférence, lorsque ladite au moins une portion vrillée est formée, la distance séparant les premières (ou secondes) portions supérieure et inférieure est augmentée ou diminuée.

Dans une variante, la première branche intermédiaire a une épaisseur e0 dans sa première zone intermédiaire qui est inférieure à l'épaisseur e1 de la première portion intermédiaire supérieure et/ou à l'épaisseur e2 de la première portion intermédiaire inférieure.

Dans des modes de réalisation, la seconde branche intermédiaire a une épaisseur e3 dans sa seconde zone intermédiaire qui est inférieure à l'épaisseur e4 de la seconde portion intermédiaire supérieure et/ou à l'épaisseur e5 de la seconde portion intermédiaire inférieure.

La première ou seconde zone intermédiaire est ainsi pour la première ou seconde branche intermédiaire une zone de faiblesse permettant le pliage des premières ou secondes portions intermédiaires supérieure et inférieure l'une vers l'autre.

Dans une variante de réalisation, le dispositif implantable comprend un second ressort latéral comprenant une seconde armature comprenant un ou plusieurs élément(s) longiligne(s) au moins en partie métallique(s).

Il s'agit du second ressort latéral décrit dans le présent texte.

Dans une variante de réalisation, le dispositif implantable comprend une armature principale comprenant la première armature et la seconde armature, et l'armature principale s'étend en partie à travers la cale intervertébrale.

Dans un mode de réalisation, la première armature et la seconde armature sont solidarisées par l'intermédiaire d'une ou plusieurs branche(s) de jonction, en particulier la ou lesdites branche(s) de jonction s'étend(ent) au moins partiellement à travers la cale intervertébrale, notamment entre les zones avant et arrière de la cale intervertébrale ou entre les première et seconde zones latérales de la cale intervertébrale. La ou lesdites branche(s) de jonction comprend/comprennent de préférence au moins un élément longiligne métallique selon l'invention.

Dans une variante de réalisation, le premier ressort latéral a une forme sensiblement de première boucle.

Dans des modes de réalisation, le second ressort latéral a une forme sensiblement de seconde boucle.

De préférence, la première boucle et/ou la seconde boucle comprend/comprennent (chacune) une zone centrale vide.

Ladite zone centrale vide facilite le passage de la position B dans la position A, et inversement, puisqu'il n'y a pas d'obstacle au rapprochement ou à l'écartement des (premières ou secondes) portions supérieure et inférieure entre-elles du premier et/ou du second ressort(s) latéral(aux). Dans une variante, la première armature, et éventuellement la seconde armature, est/sont revêtue(s) en tout ou partie d'au moins un matériau choisi parmi : les polymères, les textiles, et une combinaison de ces derniers.

De préférence, le ou les élément(s) longiligne(s) au moins en partie métalliques, notamment métallique(s), est/sont revêtu(s), en particulier surmoulé(s), d'au moins un matériau polymère, plus particulièrement choisi parmi les élastomères, tels que les silicones et les polyuréthanes.

Dans une variante, le premier ressort latéral et/ou le second ressort latéral comprend/ comprennent une ou des portion(s) qui est/sont exempte(s) d'élément longiligne au moins en partie métallique, en particulier comprenant/(constituée(s) d') au moins un matériau polymère, notamment élastomérique.

Dans une variante, le premier ressort latéral et/ou le second ressort latéral comprend/ comprennent une première et/ou une seconde branche(s) intermédiaire(s) polymérique(s), en particulier exempte(s) d'élément longiligne métallique. De préférence, la première branche intermédiaire polymérique est en liaison avec les premières branches supérieure et inférieure de la première armature, et/ou, la seconde branche intermédiaire polymérique est en liaison avec les secondes branches supérieure et inférieure de la seconde armature.

Dans une variante, la première branche supérieure, et/ou, la première branche inférieure, et/ou, la première branche intermédiaire, et/ou la première portion intermédiaire supérieure, et/ou, la première portion intermédiaire inférieure, et/ou, la seconde branche supérieure, et/ou, la seconde branche inférieure, et/ou, la seconde branche intermédiaire, et/ou la seconde portion intermédiaire supérieure, et/ou, la seconde portion intermédiaire inférieure, et/ou la ou les branche(s) de jonction, comprend/comprennent chacune au moins un élément longiligne au moins en partie métallique, éventuellement revêtu d'au moins un matériau polymère.

Dans une variante, le premier ressort latéral comprend une partie crâniale et une partie caudale, et la partie crâniale est inclinée par rapport à la partie caudale, en particulier d'un angle α1.

De préférence, ledit angle α1 est inférieur ou égal à 60°, de préférence inférieur ou égal à 50°, encore de préférence inférieur ou égal à 40°, préférentiellement supérieur ou égal à 15°.Cet agencement du premier ressort latéral permet d'assurer au dispositif implantable une bonne conformabilité anatomique dans l'espace interlamaire et dans l'espace inter-épineux voisin. Le dispositif implantable est ainsi plus stable dans sa position d'implantation.

En effet, lorsque le dispositif implantable est dans sa position d'implantation, la partie crâniale du premier ressort latéral est en butée contre la lame inférieure de la vertèbre sus-jacente ou supérieure tandis que la partie caudale du premier ressort latéral est en butée contre la lame supérieure de la vertèbre sous-jacente ou inférieure.

Le premier ressort latéral forme un organe de retenue configuré pour venir en butée contre les lames des vertèbres sus et sous-jacentes et éviter toute migration antérieure, c'est-à-dire vers l'espace dural, de la cale intervertébrale et donc du dispositif.

L'inclinaison de la partie crâniale, en particulier l'inclinaison postérieure de la partie crâniale, par rapport à la partie caudale permet avantageusement d'assurer un contact optimisé car s'adaptant à l'inclinaison naturelle formée entre la lame supérieure et la lame inférieure des vertèbres sus-jacente et sous-jacente traitées.

Le dispositif implantable est ainsi plus stable dans sa position d'implantation, et améliore ses fonctions d'appui et de maintien d'un écartement intervertébral.

On comprend dans le présent texte par partie crâniale, toute partie orientée vers la tête du sujet traité lorsque le dispositif implantable est dans sa position d'implantation.

On comprend dans le présent texte par partie caudale, toute partie éloignée de la tête, et donc orientée vers les pieds, du sujet traité lorsque le dispositif implantable est dans sa position d'implantation.

On comprend par inclinaison postérieure que ladite partie, en position d'implantation, est inclinée vers l'arrière ou le côté postérieur du sujet traité en position debout.

De préférence, l'angle α1 et/ou l'angle a2 (ci-après) et/ou l'angle β1 est/sont mesuré(s) dans le sens trigonométrique.

Dans un mode de réalisation, la partie crâniale du premier ressort latéral a sa plus grande hauteur Hcr, en particulier la hauteur de sa face antérieure, inférieure à la plus grande hauteur Hca de la partie caudale du premier ressort latéral, en particulier la hauteur de la face antérieure de la partie caudale.

On comprend par plus grande hauteur que la partie peut présenter différentes hauteurs mais que la hauteur considérée est la plus importante mesurée.

Dans un mode de réalisation, une jonction entre la partie crâniale et la partie caudale est à une distance dcr d'un plan (Tapi) passant par la zone d'appui supérieure et à une distance dca d'un plan (Tap2) passant par la zone d'appui inférieure, dcr étant inférieur à dca.

Dans une variante de réalisation, le premier ressort latéral comprend une première armature comprenant des premières branches supérieure et inférieure, et la première banche supérieure est inclinée par rapport à la première branche inférieure, en particulier dudit angle α1.

Dans une variante de réalisation, la partie crâniale du premier ressort latéral comprend la première branche supérieure, et éventuellement la première portion intermédiaire supérieure d'une première branche intermédiaire.

Dans un mode de réalisation, la partie caudale du premier ressort latéral comprend la première branche inférieure, et éventuellement la première portion intermédiaire inférieure d'une première branche intermédiaire.

Dans une variante, le second ressort latéral comprend une partie crâniale et une partie caudale, et la partie crâniale est inclinée par rapport à la partie caudale, en particulier d'un angle α2.

De préférence, ledit angle α2 est inférieur ou égal à 60°, de préférence inférieur ou égal à 50°, encore de préférence inférieur ou égal à 40°, préférentiellement supérieur ou égal à 15°.

Cet agencement du second ressort latéral permet d'assurer au dispositif implantable une bonne conformabilité anatomique dans l'espace interlamaire et dans l'espace inter-épineux voisin. Le dispositif implantable est ainsi plus stable dans sa position d'implantation.

Dans un mode de réalisation, le second ressort latéral comprend une seconde armature comprenant des secondes branches supérieure et inférieure, et la seconde banche supérieure est inclinée par rapport à la seconde branche inférieure, en particulier dudit angle α2.

Dans un mode de réalisation, la partie crâniale comprend la seconde branche supérieure, et éventuellement la seconde portion intermédiaire supérieure d'une seconde branche intermédiaire.

Dans un mode de réalisation, la partie caudale comprend la seconde branche inférieure, et éventuellement la seconde portion intermédiaire inférieure d'une seconde branche intermédiaire.

Lesdites premières branches supérieure et inférieure et/ou lesdites secondes branches inférieure et/ou supérieure peuvent comprendre/présenter l'une quelconque des caractéristiques définies dans le présent texte.

Dans une variante, les parties caudale et crâniale du premier ressort latéral comprennent une première armature revêtue au moins en partie, de préférence totalement, d'un matériau polymérique, éventuellement en combinaison avec un matériau textile.

Dans une variante, les parties caudale et crâniale du second ressort latéral comprennent une seconde armature revêtue au moins en partie, de préférence totalement, d'un matériau polymérique, éventuellement en combinaison avec un matériau textile.

Dans une variante, la partie crâniale comprend une face antérieure comprenant une région passant par un plan Pa, et la partie caudale comprend une face antérieure comprenant une région passant par un plan Pb, les plans Pa et Pb étant sécants, en particulier forment ledit angle α1 (pour le premier ressort latéral) ou l'angle α2 (pour le second ressort latéral).

Ladite partie crâniale et ladite partie caudale peuvent être celles de la première armature ou de la seconde armature.

De préférence, l'angle est mesuré dans le sens trigonométrique.

La face antérieure de la partie crâniale peut être comprise dans le plan Pa.

La face antérieure de la partie caudale peut être comprise dans le plan Pb.

Le plan Pb est de préférence sensiblement parallèle au plan frontal F.

Dans une variante, la cale intervertébrale comprend une face antérieure comprenant au moins une région passant sensiblement par un plan Pc, les plans Pb et Pc étant sécants.

La face antérieure de la cale intervertébrale peut être comprise dans le plan Pc.

De préférence, les plans Pc et Pb forment un angle β1 supérieur à 0° et inférieur ou égal à 30°, en particulier inférieur ou égal à 20°.

La cale intervertébrale comprend une face antérieure inclinée par rapport au plan Pb passant par ladite région de la face antérieure de la partie caudale du premier ressort latérale.

Dans une variante, la face antérieure de la cale intervertébrale passe par un plan Pc formant un angle θ avec un plan Tapi passant par la zone d'appui supérieure de la cale ou un plan Tap2 passant par la zone d'appui inférieure de la cale, ledit angle θ est supérieur ou égal à 30°, et inférieur ou égal à 90°, en particulier supérieur ou égal à 50°, notamment supérieur ou égal à 60°.Dans une variante, la cale intervertébrale comprend au moins un matériau polymère élastomérique, en particulier choisi parmi les silicones, ou les polyuréthanes.

Dans une variante de réalisation, la cale intervertébrale comprend une enveloppe extérieure textile, en particulier enveloppant au moins partiellement un noyau dans au moins un matériau polymère.

Ledit noyau est de préférence dans au moins un matériau polymère élastomérique, notamment choisi parmi les silicones et les polyuréthanes.

La présente invention a pour objet, selon un deuxième aspect, un ancillaire de manipulation d'un dispositif implantable selon l'une quelconque des variantes de réalisation en référence au premier aspect de l'invention. L'ancillaire comprend un dispositif de préhension et un dispositif configuré pour permettre le placement du dispositif implantable dans sa position d'introduction dans un élément allongé creux et la sortie du dispositif implantable dudit élément allongé creux.

Ledit ancillaire peut également être configuré pour aider au déploiement du dispositif implantable lors de son passage de sa position d'introduction vers sa position d'implantation. Ledit élément allongé creux est de préférence un trocart.

### Description des dessins

L'invention sera mieux comprise à la lecture de la description qui suit des modes de réalisation de l'invention donnés à titre d'exemples non limitatifs, en référence aux dessins annexés, sur lesquels :
[Fig. 1] la figure **1** illustre de manière schématique, vue en perspective, et selon sa face avant, un premier exemple de dispositif implantable selon l'invention dans la position d'implantation, les premier et second ressorts latéraux étant dans la position B;
[Fig. 2] la figure **2** illustre de manière schématique, vue en perspective, et selon sa face arrière, le dispositif implantable représenté à la figure 1;
[Fig. 3] la figure **3** illustre de manière schématique, vue de dessus, le dispositif implantable représenté aux figures 1 et 2;
[Fig. 4] la figure **4** représente de manière schématique, vue en perspective l'armature principale du dispositif implantable représenté aux figures 1 à 3;
[Fig. 5] la figure **5** représente de manière schématique, en perspective et vue selon sa face avant, le dispositif implantable représenté sur les figures 1 à 3 dans une position d'introduction dans laquelle les premier et second ressorts latéraux sont dans une première position A;
[Fig. 6] la figure **6** représente de manière schématique, en perspective et vue selon sa face avant, le dispositif implantable représenté sur les figures 1 à 3 dans une position d'introduction dans laquelle les premier et second ressorts latéraux sont dans une seconde position A;
[Fig. 7] la figure **7** représente de manière schématique, en perspective et vue selon sa face avant, le dispositif implantable représenté sur les figures 1 à 3 dans une position d'introduction dans laquelle les premier et second ressorts latéraux sont dans une troisième position A;
[Fig. 8] la figure **8** illustre de manière schématique, vue en perspective, et selon sa face avant, un second exemple de dispositif implantable selon l'invention dans la position d'implantation;
[Fig. 9] la figure **9** illustre de manière schématique, vue en perspective, de manière éclatée, et selon sa face arrière, le seconde exemple de dispositif implantable représenté à la figure 8 dans la position d'implantation;
[Fig.10] la figure **10** illustre de manière schématique vue en perspective, selon sa face avant, un troisième exemple de dispositif implantable selon l'invention dans la position d'implantation;
[Fig.11] la figure **11** illustre de manière schématique, vue en perspective, et selon sa face avant, un quatrième exemple de dispositif implantable selon l'invention dans la position d'implantation;
[Fig.12] la figure **12** illustre de manière schématique, vue en perspective, et selon sa face avant, un cinquième exemple de dispositif implantable selon l'invention;
[Fig.13] la figure **13** illustre de manière schématique, vue en perspective, et selon sa face avant, un sixième exemple de dispositif implantable selon l'invention;
[Fig.14] la figure **14** illustre de manière schématique, vue en perspective, et selon sa face arrière, le sixième exemple de dispositif implantable représenté à la figure 13;
[Fig.15] la figure **15** illustre de manière schématique, en perspective, un septième exemple de dispositif implantable selon l'invention positionné sur un niveau vertébral à traiter;
[Fig.16] la figure **16** illustre de manière schématique, sensiblement selon sa face antérieure, un huitième exemple de dispositif implantable;
[Fig.17] la figure **17** illustre de manière schématique, vue de côté, le dispositif implantable représenté à la figure 16 dans une position correspondant à sa position d'implantation;
[Fig.18] la figure **18** illustre de manière schématique, vue de côté et selon un plan de coupe sagittal, le dispositif implantable représenté sur les figures 16 et 17 positionné sur un niveau vertébral à traiter sans les articulations postérieures; et
[Fig.19] la figure **19** illustre de manière schématique, vue de côté, le dispositif implantable selon l'invention positionné sur un niveau vertébral à traiter.

### Description des modes de réalisation

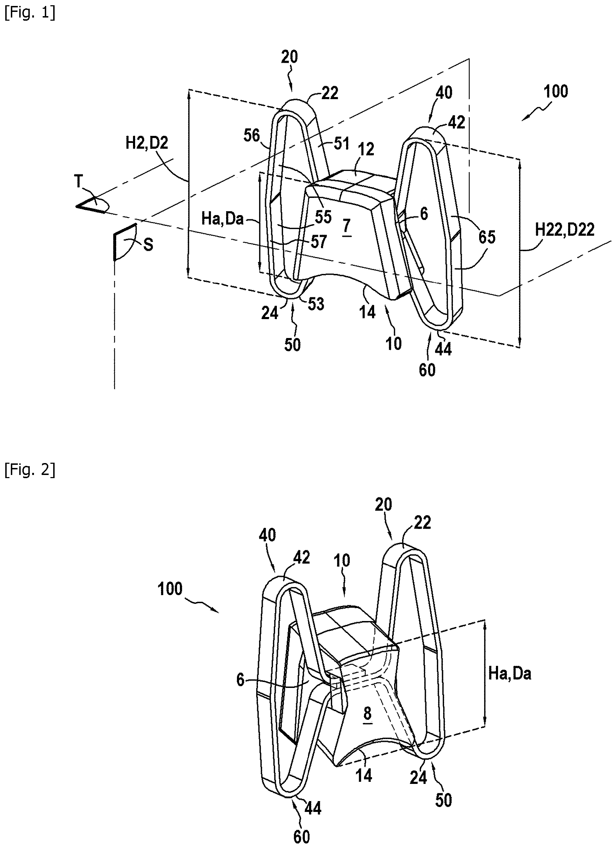

Le premier exemple de dispositif implantable 100 représenté sur les figures 1 à 7, notamment pour maintenir un écartement intervertébral entre des vertèbres sus et sous-jacentes, comprend une cale intervertébrale 10 comprenant une zone supérieure d'appui 12, en particulier sur la lame de la vertèbre sus-jacente, et une zone inférieure d'appui 14, en particulier sur la lame de la vertèbre sous-jacente, distantes d'une distance Da minimum pour le maintien d'un écart intervertébral. Le dispositif 100 comprend un premier ressort latéral 20 ayant une hauteur H1 dans une position A correspondant à la position d'introduction du dispositif 100, et une hauteur H2 dans une position B correspondant à une position d'implantation du dispositif 100, H2 étant différente de H1. Le dispositif 100 comprend également un second ressort latéral 40 ayant une hauteur H11 dans une position A correspondant à une position d'introduction du dispositif 100, et une hauteur H22 dans une position B, correspondant à une position d'implantation du dispositif 100, H22 étant différente de H11. Le premier ressort latéral 20 et le second ressort latéral 40 sont chacun configurés pour passer de la position A à la position B librement sous l'effet de leurs élasticités intrinsèques, et former chacun un organe de retenue agencé par rapport à la cale intervertébrale 10 pour bloquer la migration de la cale intervertébrale 10 vers le canal rachidien dans la position B.

Le premier ressort latéral 20 comprend des premières portions supérieure 22 et inférieure 24 distantes de D1 dans la position A, et distantes de D2 dans la position B, D2 étant différente de D1. Le second ressort latéral 40 comprend des premières portions supérieure 42 et inférieure 44 distantes de D11 dans la position A, et distantes de D22 dans la position B, D22 étant différente de D11.

La cale intervertébrale 10 a une hauteur maximum Ha, inférieure à H2 et H22 dans la position d'implantation du dispositif 100.

Les premier et second ressorts latéraux 20,40 s'étendent de part et d'autre du plan coupe sagittale S de la cale intervertébrale 10.

Les premières portions supérieure 22 et inférieure 24 du premier ressort latéral 20 se projettent de part et d'autre d'un plan de coupe transversal T de la cale intervertébrale. De plus, les secondes portions supérieure 42 et inférieure 44 du second ressort latéral 40 se projettent également de part et d'autre du plan de coupe transversal T de la cale intervertébrale 10.

La cale intervertébrale 10 comprend des première et seconde zones latérales 5,6, notamment disposées de part et d'autre du plan de coupe sagittal S de la cale intervertébrale 10. La cale intervertébrale 10 comprend également des zones avant et arrière 7,8 disposées de part et d'autre du plan de coupe frontal F de la cale intervertébrale 10.

Le premier ressort latéral 20 comprend une première armature 50 et le second ressort latéral 40 comprend une seconde armature 60.

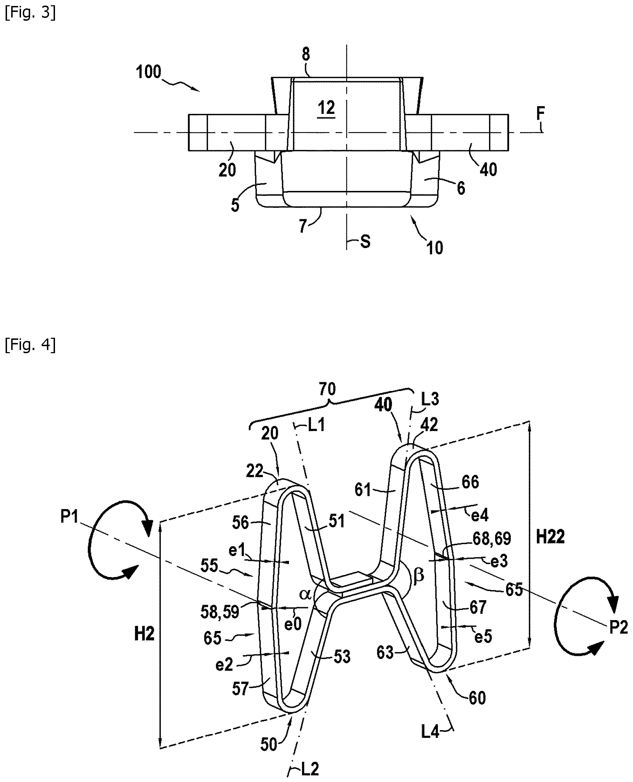

Les première et seconde armatures 50,60 forment une armature principale 70, représentée seule à la figure 4, traversant la cale intervertébrale 10, de sa première zone latérale 5 vers sa seconde zone latérale 6. L'armature principale 70 comprend dans cet exemple précis un seul élément longiligne 65 notamment métallique, tel un ruban. L'élément longiligne 65 est de préférence en Nitinol. La composition de l'alliage est par exemple déterminée en sorte que la température d'As soit de l'ordre de 17°C, ainsi à température ambiante, par exemple de l'ordre de 20°C, l'armature principale 70 est dans un état super-élastique.

La première armature 50 comprend des premières branches supérieure 51 et inférieure 53 s'étendant de la première zone latérale 5 en divergent l'une de l'autre, ainsi qu'une première branche intermédiaire 55 en liaison à ses extrémités avec les premières branches supérieure 51 et inférieure 53. La première branche intermédiaire 55 comprend des premières portions intermédiaires supérieure 56 et inférieure 57 convergent vers une première zone intermédiaire 58, dans cet exemple précis, une zone de pliage, et autour de laquelle lesdites premières portions intermédiaires supérieure 56 et inférieure 57 pivotent, en particulier autour de l'axe P1, lors du passage de la position A vers la position B, et inversement.

La première branche supérieure 51 passe par un axe L1 et la première branche inférieure 53 passe par un axe L2, L1 et L2 définissent entre-eux un angle α supérieur à 90° dans la position B représentée notamment à la figure 4.

La seconde branche supérieure 66 passe par un axe L3 et la seconde branche inférieure 63 passe par un axe L4, L3 et L4 définissent entre-eux un angle β supérieur à 90°.

La seconde armature 60 comprend des secondes branches supérieure 61 et inférieure 63 s'étendant de la seconde zone latérale 6 en divergent l'une de l'autre, ainsi qu'une seconde branche intermédiaire 65 en liaison à ses extrémités avec les secondes branches supérieure 61 et inférieure 63. La seconde branche intermédiaire 65 comprend des secondes portions intermédiaires supérieure 66 et inférieure 67 convergent vers une seconde zone intermédiaire 68, en particulier une zone de pliage, et autour de laquelle lesdites secondes portions intermédiaires supérieure 66 et inférieure 67 pivotent, en particulier autour de l'axe P2, lors du passage de la position A vers la position B.

Les première et seconde armatures 50 et 60 sont chacune sous forme de boucle, telles des ailes, en particulier comprenant un espace vide centrale facilitant les déformations des premier et second ressorts 20 et 40.

Les première et seconde zones intermédiaires 58,68 sont, dans cet exemple précis, des zones de pliage 59,69 sensiblement centrée respectivement sur les première et seconde branches intermédiaires 55,65. La zone de pliage 59 a une épaisseur e0 inférieure à l'épaisseur e1 de la première portion intermédiaire supérieure 56 et à l'épaisseur e2 de la première portion intermédiaire inférieure 57. La zone de pliage 69 a une épaisseur e3 inférieure à l'épaisseur e4 de la seconde portion intermédiaire supérieure 66 et à l'épaisseur e5 de la seconde portion intermédiaire inférieure 67.

Dans cet exemple précis, et éventuellement en combinaison avec différentes variantes de l'invention, les première et second branches intermédiaires 55,65 sont en outre des lames ressorts.

En fonctionnement, le dispositif implantable 100 est disposé dans sa position d'introduction, en exerçant une contrainte mécanique permettant le rapprochement ou l'écartement des premières et secondes portions supérieures 22,42 et inférieures 24,44.

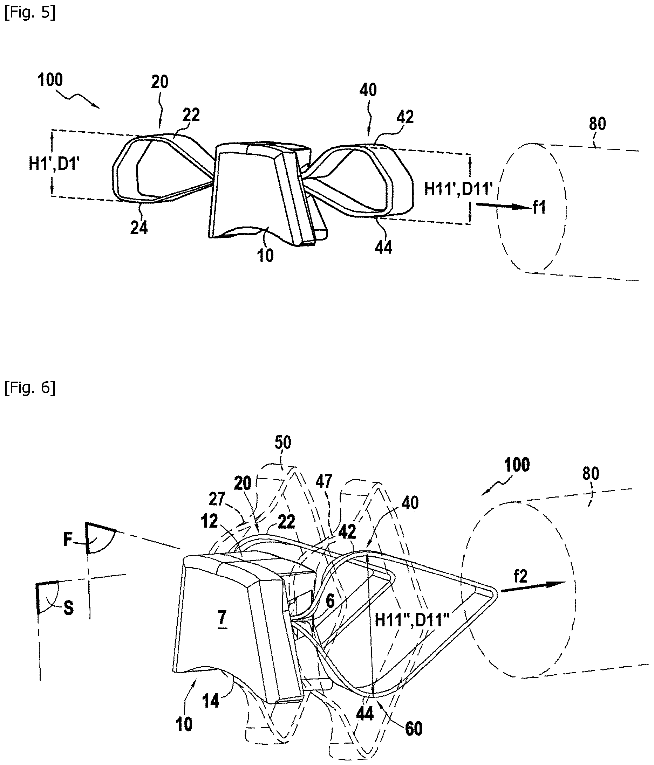

Dans une première position A représentée à la figure 5, le dispositif implantable 100 peut être disposé dans un tube 80 selon la flèche f1, par exemple un trocart, dont le diamètre intérieur est inférieur à H2 et H22, en sorte que les premier et second ressorts latéraux 20 et 40 aient des hauteurs respectivement H1' et H11' réduites par rapport aux hauteurs H2 et H22. De même, les distances séparant les premières et secondes portions supérieures et inférieures (22,24 ; 42,44) sont réduites respectivement selon les distances D1' et D11'. Dans ce cas, la cale intervertébrale 10 est d'abord insérée selon sa première ou seconde face latérale 5 ou 6.

Dans une seconde position A représentée à la figure 6, les premier et second ressorts latéraux 20 et 40 sont rabattus contre les zones latérales respectivement 5 et 6 de la cale intervertébrale 10. Les premier et second ressorts latéraux 20 et 40, disposés respectivement dans des plans frontaux F1 et F2 (non représentés) dans la position B, sensiblement parallèles au plan frontal F, sont déformés pour être disposés respectivement dans des plans sagittaux S1 et S2 (non représentés) dans la seconde position A, les plans S1 et S2 étant sensiblement parallèles au plan sagittal S. Le dispositif implantable 100 peut alors être inséré dans le trocart 80 selon le sens de la flèche f2. Dans cas, la cale intervertébrale 10 est d'abord insérée par sa face avant 7 ou sa face arrière 8 dans le trocart 80. Les premières et secondes portions supérieures et inférieures de chacun des premier et second ressorts latéraux 20,40 sont rapprochées entraînant une diminution des hauteurs H2 et H22 à H1" (non représenté) et H11", et donc une diminution des distances D2 et D22 à D1" (non représentée) et D11". Dans cette position A, les premier et second ressorts 20 et 40 comprennent chacun une portion d'élément longiligne 65 vrillée respectivement 27 et 47.

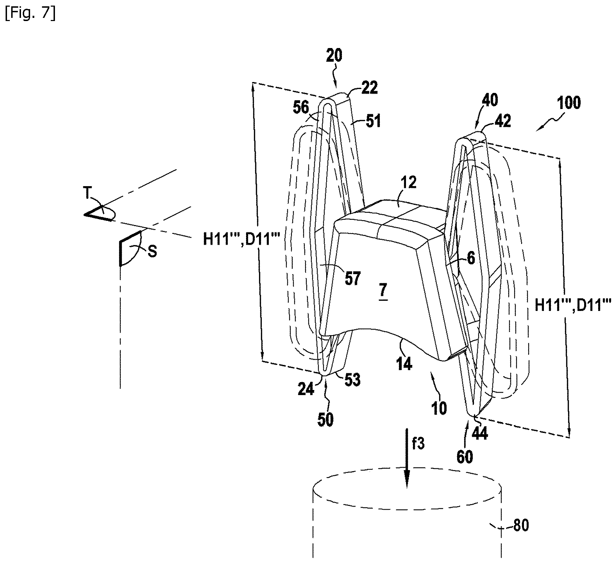

Dans une troisième position A représentée à la figure 7, le dispositif implantable 100 est disposé dans un tube 80 selon le sens d'insertion correspondant à la flèche f3. Le tube 80 est dans cet exemple précis un trocart dont le diamètre intérieur est déterminé en sorte qu'il soit inférieur à l'encombrement du dispositif implantable 100 dans la position d'implantation et considéré selon le sens d'insertion f3. Dans ce cas, la cale intervertébrale 10 est d'abord insérée dans le trocart par sa face inférieure 14 ou supérieure 12. Les premières et secondes portions supérieures et inférieures (22,24 ; 42,44) des ressorts (20,40) sont écartées en sorte que les premier et second ressorts latéraux 20 et 40 aient des hauteurs respectivement H1‴ et H11‴ augmentées par rapport aux hauteurs H2 et H22. De même, les distances séparant les premières et secondes portions supérieures et inférieures (22,24 ; 42,44) sont augmentées à des distances D1‴ et D11‴.

Pour les trois premières positions A représentées schématiquement sur les figures 5 à 7, l'encombrement du dispositif 100 étant réduit dans au moins une direction f1, f2 ou f3, ce dernier est acheminé sur le niveau vertébral à traiter en étant amené de l'extrémité proximale jusqu'à l'extrémité distale du trocart 80. Une fois libéré sur le site opératoire, le dispositif implantable 100 se positionne dans la position d'implantation, sous l'effet de la température corporelle activant la mémoire de la forme d'éducation de l'armature principale 70 correspondant à la position B, et/ou sous l'effet des lames ressort des première et seconde branches intermédiaires 55,65 accompagnant ce déploiement. Les premier et second ressorts latéraux 20,40 passent ainsi de la première/seconde/troisième position A à la position B librement, c'est-à-dire du fait de leurs élasticités intrinsèques et non par l'intermédiaire d'un dispositif de serrage et/ou de déformation maintenant en position B lesdits ressorts 20,40.

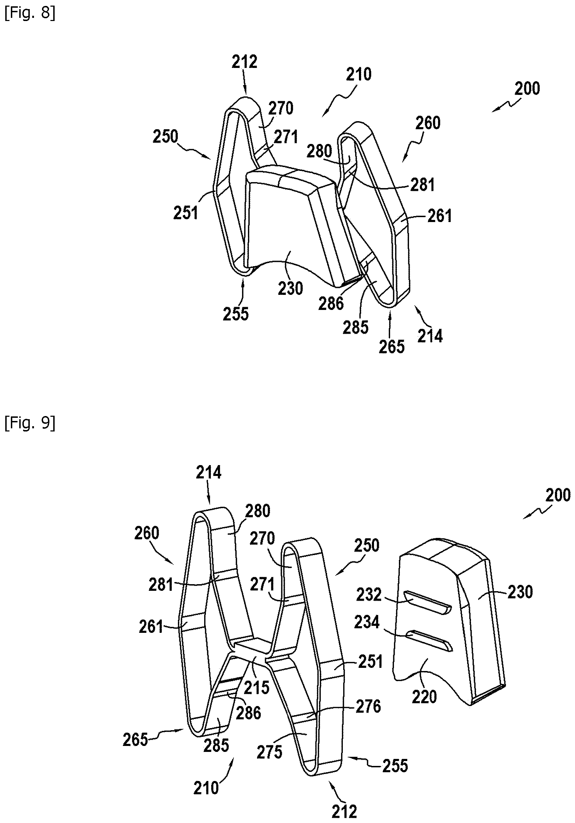

Le second dispositif implantable 200 représenté sur les figures 8 et 9 dans sa position d'implantation diffère du dispositif 100 en ce que son armature principale 210 est solidarisée à la face arrière 220 de la cale intervertébrale 230 par l'intermédiaire des protrusions 232,234. La cale intervertébrale 230 est ainsi surmoulée sur l'armature principale 210. Lors du surmoulage, les protrusions 232 et 234 de solidarisation de la cale intervertébrale 230 à l'armature 210 sont formées. De préférence, la cale intervertébrale 220 moulée comprend au moins un matériau polymère élastomérique, en particulier choisi parmi les silicones et les polyuréthanes.

L'armature principale 210 comprend des premier et second ressorts latéraux 212 et 214 dans un élément longiligne métallique, en particulier à mémoire de forme (par ex. nitinol). Ces premier et second ressorts 212,214 comprennent des zones d'épaisseur réduite ou zones de pliage, notamment formant ainsi des lames ressort. L'armature principale 210 comprend des première et seconde branches intermédiaires 250 et 260 comprenant chacune une zone d'épaisseur réduite ou de pliage respectivement 251 et 261.

L'armature principale 210 comprend également des premières branches supérieure 270 et inférieure 275 comprenant les zones d'épaisseur réduite ou de pliage 271 et 276. De même, l'armature principale 210 comprend des secondes branches supérieure 280 et inférieure 285 comprenant chacune une zone d'épaisseur réduite ou de pliage respectivement 281 et 286. Ces zones de pliage 251,261,271, 276,281,286 facilitent la déformation des premier et second ressorts 212,214 pour le rapprochement ou l'écartement de leurs premières et secondes portions supérieures et inférieures, et le retour élastique de l'armature 210 lorsque la contrainte mécanique n'est plus appliquée. A l'exception, des zones de pliage additionnelles définies ci-dessus, l'armature principale 210 est similaire à l'armature 70. Les premières branches supérieure et inférieure 270,275 et la première branche intermédiaire 250, forment une première armature 255. Les secondes branches supérieure 280 et inférieure 285, et la seconde branche intermédiaire 260, forment une seconde armature 265. Les première et seconde armatures 255,265 sont solidarisées au niveau d'une branche de jonction 215. Les première et seconde ressorts latéraux 212 et 214, notamment leurs armatures respectives 255 et 265, sont chacune sous forme de boucle, telles des ailes, en particulier comprenant un espace vide centrale facilitant les déformations des premier et second ressorts 212 et 214. Dans ce cas précis, l'armature principale 210 est formé d'un seul élément longiligne métallique, de sorte que la branche de jonction 215 comprend les extrémités libres superposées dudit élément longiligne replié sur lui-même. Lesdites extrémités de l'élément longiligne peuvent être maintenues ensemble par collage, soudure et/ou par les protrusions 232 et 234, ou encore par une partie surmoulée de la cale intervertébrale 230.

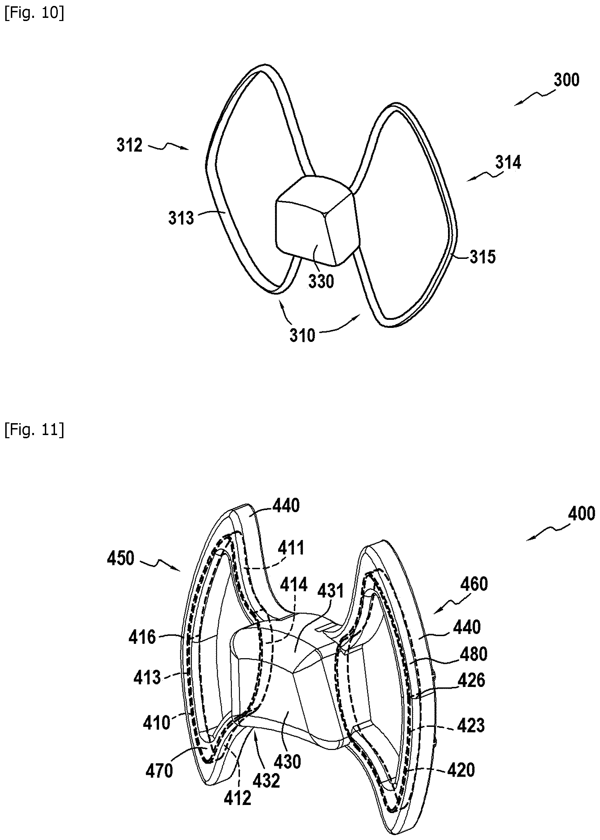

Le troisième exemple de dispositif implantable 300 représentée à la figure 10 comprend une cale intervertébrale 330 et une armature principale 310 comprenant un premier ressort latéral 312 et un second ressort latéral 314 représentés dans la position B. Dans cet exemple précis, l'armature principale 310 comprend deux éléments longilignes métalliques 313 et 315 distincts, par exemple en nitinol, formant respectivement le premier ressort latéral 312 et le second ressort latéral 314. En particulier, les éléments longilignes 313 et 315 sont chacun sous forme de boucle. La cale intervertébrale 330 est de préférence surmoulée sur les éléments longilignes 313 et 315. Le premier ressort latéral 312 comprend une première armature formée de l'élément longiligne 313, et le second ressort latéral 314 comprend une seconde armature formée de l'élément longiligne 315. Le fonctionnement du dispositif implantable 300 est similaire à celui des dispositifs implantables 100 ou 200.

Le dispositif implantable 400 représenté à la figure 11 comprend une cale intervertébrale 430 ainsi qu'une première armature 410 et une seconde armature 420 comprenant respectivement les éléments longilignes (470,480). La première armature 410 comprend des premières branches supérieure 411 et inférieure 412 ainsi qu'une première branche intermédiaire 413 en liaison à ses extrémités avec les premières branches supérieure 411 et inférieure 412. La seconde armature 420 a une construction similaire à la première armature 410. Les première et seconde armatures 410 et 420 forment une armature principale du dispositif implantable 400 mais sont indépendantes l'une de l'autre. Les premières branches supérieure 411 et inférieure 412 sont en liaison via une branche de jonction 414 traversant la cale intervertébrale 430, en particulier de la zone supérieure d'appui 431 vers la zone inférieure d'appui 432. Dans cet exemple précis, les première et seconde armatures 410 et 420 sont surmoulées par un revêtement polymère 440, en particulier élastomérique. Ce revêtement polymère épouse les armatures 410 et 420. Les première et seconde armatures 410,420 peuvent être surmoulées dans une première étape pour former le revêtement polymère, puis la cale intervertébrale 430 peut être surmoulée dans une seconde étape autour de certaines parties des premier et second ressorts latéraux (450,460) afin de les solidariser à la cale intervertébrale 430. La cale intervertébrale 430 et le revêtement polymère 440 peuvent être de manière alternative moulée en même temps dans une seule étape. Le revêtement polymère 440 protège les tissus environnants de la zone d'implantation des armatures métalliques 410,420. Le premier ressort 450 comprend ainsi la première armature 410 revêtue d'au moins un polymère, et le second ressort 460 comprend la seconde armature 420 revêtue d'au moins un polymère. Les premier et second ressorts 450 et 460 sont dans cet exemple précis sous forme de boucle. La première branche intermédiaire 413 peut comprendre une zone intermédiaire 416 qui est soit une zone de pliage telle que représentée à la figure 11 (notamment avec une épaisseur réduite de l'élément longiligne 470) facilitant le rapprochement des premières portions supérieure et inférieure d'une part, ou soit une zone d'interruption de l'armature 410 dans laquelle les extrémités libres des premières portions intermédiaires supérieure et inférieure débouchent (non représentée). La seconde branche intermédiaire 423 peut être agencée de la même façon que la première branche intermédiaire 416 par l'intermédiaire de la zone intermédiaire 426.

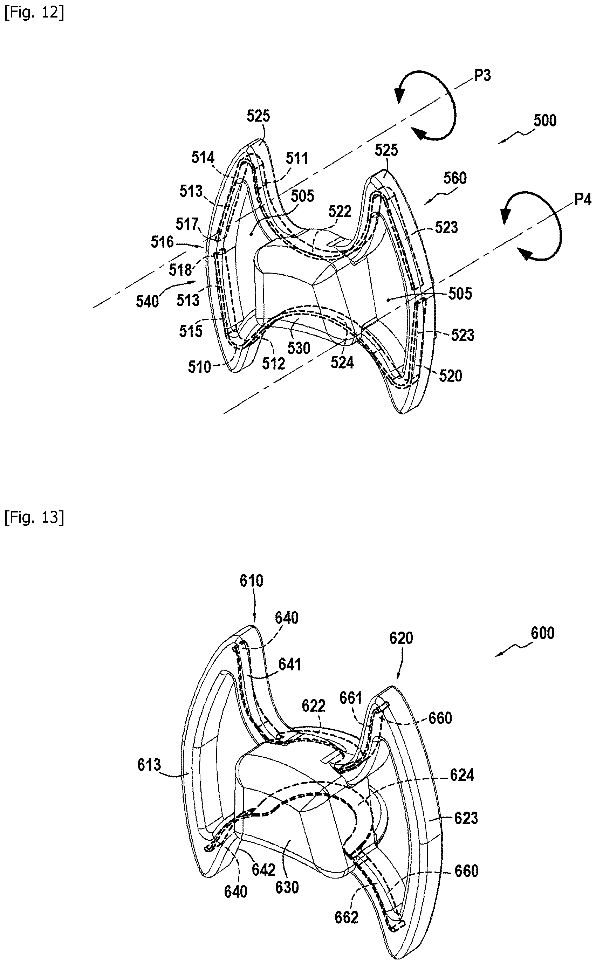

Le dispositif implantable 500 représenté à la figure 12 comprend dans sa position d'implantation un premier ressort latéral 540 et un second ressort latéral 560. Le premier ressort latéral 540 comprend une première armature 510 comprenant des premières branches supérieure 511 et inférieure 512 en liaison à leurs extrémités avec une première branche intermédiaire 513. La première branche intermédiaire 513 comprend des premières portions intermédiaires supérieure 514 et inférieure 515 convergent vers une zone intermédiaire 516 autour de laquelle lesdites premières portions intermédiaires supérieure 514 et inférieure 515 pivotent lors du passage de la position d'introduction vers la position d'implantation, en particulier autour de l'axe P3. Les premières portions intermédiaires supérieure 514 et inférieure 515 comprennent chacune une extrémité libre 517 et 518. Le second ressort latéral 560 comprend une seconde armature 520 similaire à la première armature 510. Les première et seconde armatures 510,520 forment une armature principale comprenant deux éléments longilignes métalliques distincts, par exemple en acier inoxydable élastique ou en nitinol. La première armature 510 est en liaison avec la seconde armature 520 selon deux branches de jonctions 522,524 traversant la cale intervertébrale 530 de sa première zone latérale vers sa seconde zone latérale. Dans cet exemple précis, les première et seconde armatures 510,520 sont revêtues d'un revêtement 525 comprenant au moins un matériau polymère. Ce revêtement peut être remplacé par un matériau textile ou une combinaison d'au moins un matériau textile avec au moins un matériau polymère (sous la forme d'une enduction ou d'une imprégnation ou d'un film ou d'au moins un polymère surmoulé sur le matériau textile). Les premier et second ressorts latéraux 540 et 560 ont chacun une forme générale de boucle avec une région interne vide 505. Le revêtement 525 dans au moins un matériau polymère revêt également la zone intermédiaire 516 et fait la jonction entre les extrémités libres 517,518 de sorte que la première branche intermédiaire 513 bien qu'étant discontinue est revêtue d'un revêtement continu. De préférence, le matériau polymère est élastomérique, c'est-à-dire qu'il présente une élasticité importante n'entravant pas le pivotement des premières portions intermédiaires supérieure 514 et 515 l'une vers l'autre. Le dispositif implantable 500 peut également ne pas comprendre de première branche intermédiaire 513, ni de seconde branche intermédiaire 523. Dans ce cas, les premier et second ressort latéraux 540 et 560 se présentent toujours sous forme de boucle mais comprennent des première et seconde branches intermédiaires polymériques exemptes d'armature et donc d'élément longiligne métallique.

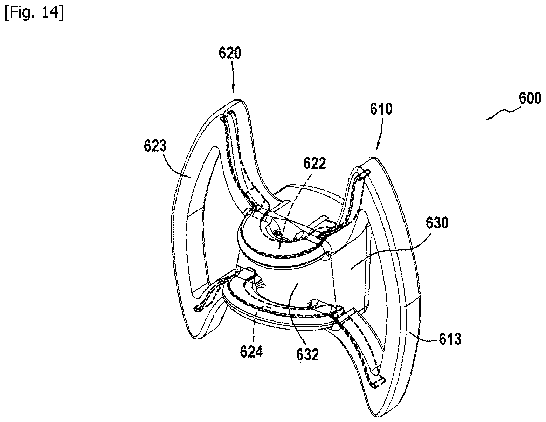

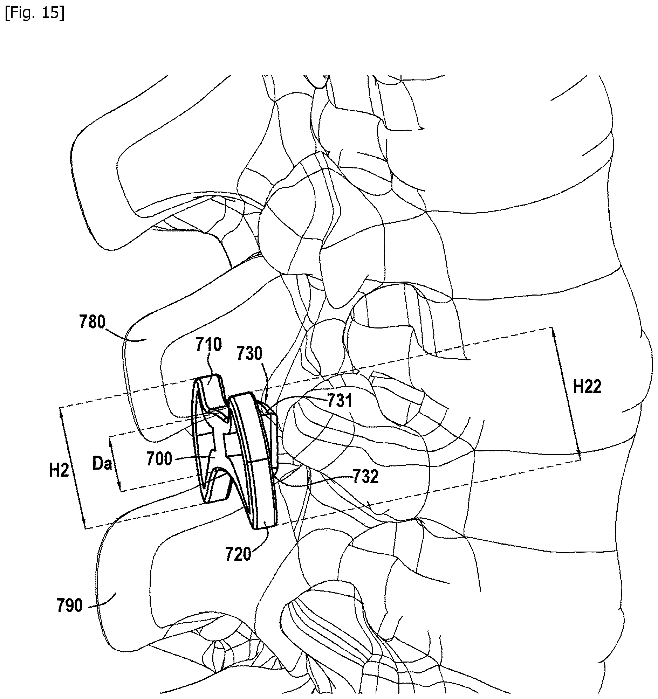

Le dispositif implantable 600, représenté sur les figures 13 et 14, dans sa position d'implantation, comprend une cale intervertébrale 630 et des premier et second ressort latéraux 610 et 620 comprenant chacun respectivement une première armature 640 et une seconde armature 660. La première armature 640 comprend des premières branches supérieure 641 et inférieure 642. La seconde armature 660 comprend des secondes branches supérieure 661 et inférieure 662. La première armature 640 est en liaison avec la seconde armature 660 par l'intermédiaire de deux branches de jonction supérieure 622 et inférieure 624 se projetant de la zone arrière 632 de la cale intervertébrale 630 en formant sensiblement chacune un arc de cercle. L'armature principale du dispositif 600 comprend ainsi les première et seconde armatures 610 et 620 ainsi que les branches de jonction supérieure 622 et inférieure 624. Les premier et second ressorts latéraux 610 et 620 ont une forme générale chacun sensiblement de boucle, telles des ailes. Le premier ressort latéral 610 comprend une première branche intermédiaire polymérique 613 ne comprenant pas d'élément longiligne métallique. De même, le second ressort latéral 620 comprend une seconde branche intermédiaire polymérique 623 ne comprenant pas d'élément longiligne métallique. De préférence, l'armature principale est revêtue, notamment par surmoulage, d'au moins un matériau polymère élastomérique. Lors du surmoulage, les première et second branches intermédiaires polymériques 613 et 623 sont moulées. Ces branches 613 et 623 contribuent à la stabilisation et au déploiement des premier et second ressorts latéraux 610 et 620. Les branches intermédiaires polymériques 613 et 623 sont moins rigides que des branches intermédiaires métalliques (telles que celles des première et seconde armatures selon l'invention), permettant ainsi de diminuer très sensiblement la constante de raideur des premier et second ressorts. Le passage de la position d'implantation à la position d'introduction pour l'insertion du dispositif implantable dans le ou les trocarts est relativement plus facile. De plus, en position d'implantation, si un contact se produit entre d'une part les portions supérieures et inférieure du premier ou second ressort latéral, et d'autre part les parties osseuses du rachis environnants faisant suite à un mouvement en extension du rachis, le premier ou second ressort se déforme facilement et ne bloque pas les mouvements du rachis. Le dispositif implantable 700 représenté à la figure 15 dans sa position d'implantation comprend une cale intervertébrale 730 et des premier et second ressorts latéraux 710 et 720. La cale intervertébrale 730 comprend une zone supérieure d'appui 731 sur une portion de la lame de la vertèbre sus jacente 780 et une zone inférieure d'appui 732 sur une portion de la lame de la vertèbre sous-jacente 790. La cale intervertébrale 730 permet de maintenir un écartement intervertébral d'au moins Da minimum correspondant à la distance séparant la zone supérieure d'appui 731 de la zone inférieure d'appui 732. Les premier et second ressorts latéraux 710 et 720 ont des hauteurs dans la position d'implantation respectivement H2 et H22 supérieures à Da, et en particulier se projettent de part et d'autre des zones supérieure et inférieure d'appui 731 et 732. Cet agencement particulier permet aux premier et second ressorts latéraux 710 et 720 de faire office également d'organes de retenue empêchant la migration de la cale intervertébrale 730 vers le canal rachidien.

De préférence, et tel que cela est visible sur les figures, les premiers et seconds ressorts latéraux des dispositifs implantables 100 à 600 sont agencés de manière similaire dans la position d'implantation aux ressorts latéraux 710 et 720 afin de faire office d'organes de retenue empêchant la migration de la cale intervertébrale vers le canal rachidien.

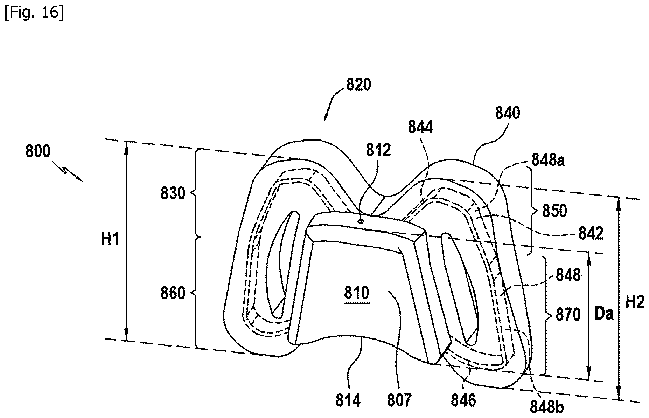

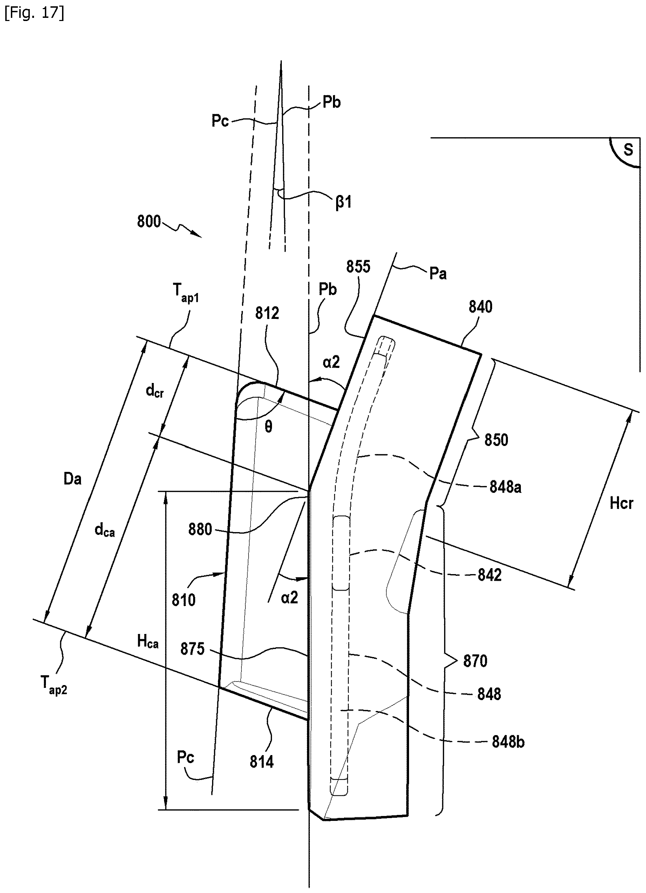

Le dispositif implantable 800 représenté sur les figures 16 à 19 dans une position d'implantation B comprend une cale intervertébrale 810 comprenant une zone supérieure d'appui 812 et une zone inférieure d'appui 814 distantes de Da. Le dispositif 800 comprend un premier ressort latéral 820 et un second ressort latéral 840 formant chacun un organe de retenue pour bloquer la migration de la cale vers l'avant en position implantée. Les premier et second ressorts latéraux ont des hauteurs H1 et H2 respectivement. Les premier et second ressorts latéraux 820,840 comprennent des parties crâniales 830,850 et des parties caudales 860,870. La partie crâniale 830 est inclinée par rapport à la partie caudale 860, en particulièrement postérieurement, en sorte de former un angle α1 (non représenté) inférieur à 60°, par exemple de l'ordre de 20°. De même, la partie crâniale 850 est inclinée par rapport à la partie caudale 870, en particulièrement postérieurement, en sorte de former un angle α2 inférieur à 60°, par exemple de l'ordre de 20° tel que représenté sur la figure 17. Le second ressort latéral 840 comprend une seconde armature 842 comprenant une seconde branche supérieure 844, une seconde branche inférieure 846, et une seconde branche intermédiaire 848. La seconde branche intermédiaire 848 comprend une seconde portion supérieure de branche intermédiaire 848a et une seconde portion inférieure de branche intermédiaire 848b. La partie crâniale 850 comprend ainsi la seconde branche supérieure 844 et la seconde portion supérieure de banche intermédiaire 848a. La partie caudale 870 comprend la seconde branche inférieure 846 et la seconde portion inférieure de branche intermédiaire 848b. Les parties crâniale 830 et caudale 860 sont agencées de manière similaire par rapport à la première armature du premier ressort latéral 820. La face antérieure 807 de la cale 810 passe par le plan Pc. Tel que cela est visible sur la figure 17, la face antérieure 855 de la partie crâniale 850 passe par le plan Pa et la face antérieure 875 de la partie caudale 870 passe par le plan Pb, les plans Pa et Pb sont sécants et forment ledit angle α2. Le même raisonnement s'applique pour les parties caudale 860 et crâniale 830 du premier ressort latéral 820.

Les plans Pb et Pc sont également sécants et forme un angle β1 inférieur ou égal à 50°, en particulier inférieur ou égal à 40°, par exemple de l'ordre de 5-6°.

La face antérieure 855 de la cale intervertébrale 810 passe par le plan Pc formant un angle θ avec un plan Tapi passant par la zone d'appui supérieure 812 de la cale ou un plan Tap2 passant par la zone d'appui inférieure 814 de la cale, ledit angle θ est supérieur ou égal à 60°.

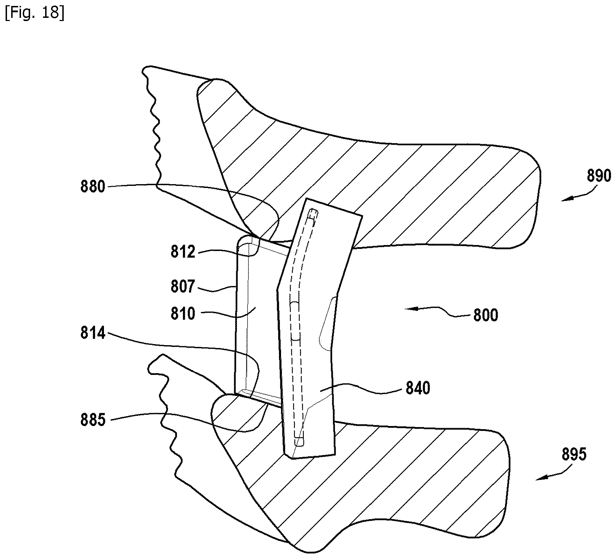

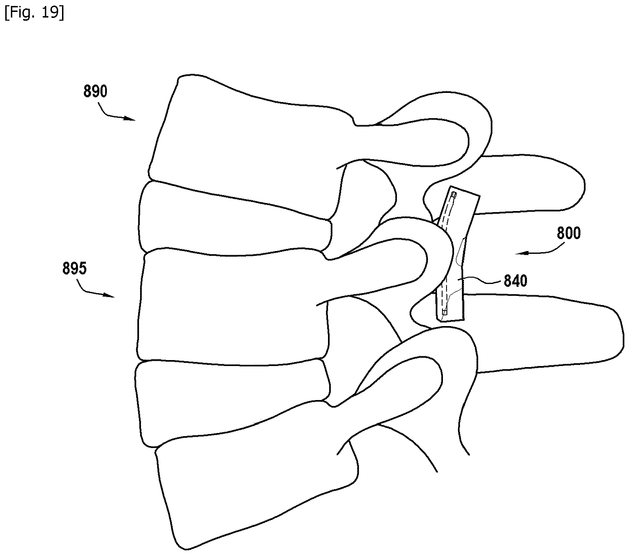

Enfin, la jonction 880 entre la partie crâniale 850 et la partie caudale 870 est distante de dcr du plan Tapi passant par la zone supérieure d'appui 812 et distante de dca du plan d'appui Tap2 passant par la zone inférieure d'appui 814, avec dcr inférieur à dca. On retrouve ainsi que la plus grande hauteur Hca de la partie caudale 870, en particulier la hauteur de la face antérieure 855, est supérieure à la plus grande hauteur Hcr, en particulier la hauteur de la face antérieure 875, de la partie crâniale 850. L'inclinaison d'un angle α2 se retrouve entre la seconde portion inférieure 848b et la seconde portion supérieure 848a, mais également entre la seconde branche supérieure 844 et la seconde branche inférieure 846. Cette inclinaison des ressorts 820 et 840 permet au dispositif d'épouser les zones du rachis à soutenir, d'améliorer ainsi le soutien mais également la stabilité. Tel que représenté sur la figure 18, les zones supérieure et inférieure d'appui 812,814 viennent en appui contre les lames sus-jacente 880 et sous-jacente 885. Les parties crâniales 830,850 viennent en butée contre la lame de la vertèbre sus-jacente 890 et les parties caudales viennent en butée contre la lame de la vertèbre sous-jacente 895 telles que représentées sur les figures 18 et 19. La combinaison de la butée, de la forme anatomique des premier et second ressorts latéraux 820,840 améliore la stabilité et le maintien d'un écart intervertébral durable.

Les dispositifs implantables 200,300,400,500,600,700 et 800 peuvent adopter indifféremment l'une des trois premières positions A illustrées sur les figures 5 à 7 pour l'introduction de ces dispositifs dans un trocart.

Les cales intervertébrales des dispositifs implantables 100 à 800 peuvent être revêtues au moins partiellement d'une enveloppe textile, en particulier la zone supérieure d'appui et/ou la zone inférieure d'appui et/ou la zone avant et/ou la zone arrière et/ou la première zone latérale et/ou la seconde zone latérale peut/peuvent être recouverte(s) au moins partiellement d'une enveloppe textile. Cette disposition a pour avantage de déclencher une colonisation des tissus environnants via la porosité du textile. Cette colonisation se propage à la surface du textile et dans l'épaisseur du textile. Une fixation mécanique complémentaire entre le dispositif implantable et les tissus environnants est ipso facto créée ce qui permet de limiter la migration du dispositif implantable vers le canal rachidien.

Les cales intervertébrales des dispositifs 100 à 800 sont de préférence fabriquées par surmoulage des armatures principales, et sont de préférence dans un élastomère choisi parmi les silicones et les polyuréthanes.

On comprend que les dimensions du dispositif implantable selon l'invention (telles que les hauteurs H1',H1",H1‴,H2,H22,Da,Ha) ainsi que celles du trocart, sont déterminées selon l'application précise, et la déformation désirée des premier et second ressorts latéraux.

## Revendications

1. Dispositif implantable (100,200,300,400,500,600,700,800), notamment, pour maintenir un écartement intervertébral entre des vertèbres sus et sous-jacentes (780,790), comprenant:
- une cale intervertébrale (10,230,330,430,530,630,730,810) comprenant une zone supérieure d'appui (12,731,812), en particulier sur au moins une portion de la lame de la vertèbre sus-jacente, et une zone inférieure d'appui (14,732,814), en particulier sur au moins une portion de la lame de la vertèbre sous-jacente, distantes d'une distance Da minimum pour le maintien d'un écart intervertébral, et
- un premier ressort latéral (20,212,312,450,540,640,820), et un second ressort latéral, ayant chacun une position A par rapport à la cale intervertébrale (10,230,330,430,530,630,730,810) dans une position d'introduction du dispositif implantable (100,200,300,400,500,600,700,800), et une position B par rapport à la cale intervertébrale (10,230,330,430,530,630,730,810,) dans une position d'implantation du dispositif implantable, les positions A et B étant différentes, et en ce que le premier ressort latéral (20,212,312,450,540,640,820), et le second ressort latéral, sont configuré(s) :
- pour passer de la position A à la position B librement, et
- former des organes de retenue agencés par rapport à la cale intervertébrale (10,230,330,430,530,630,730,810) pour bloquer la migration de la cale intervertébrale (10,230,330,430,530,630,730,810) vers le canal rachidien au moins dans la position B, **caractérisé en ce que :**
- le premier ressort latéral (20,212,312,450,540,640,820) comprend une première armature (50,255,410,510,640,820) comprenant un ou plusieurs élément(s) longiligne(s) au moins en partie métallique(s) (65,313,315,470,480), **en ce que** la première armature (50,255,410,510,640,820) comprend une première branche supérieure (51,270,411,511,641) et une première branche inférieure (53,275,412,512,642) qui divergent l'une de l'autre, et **en ce que** la première armature (50,255,410,510) comprend une première branche intermédiaire (55,250,413,513), continue ou discontinue, en liaison avec la première branche supérieure (51,270,411,511) et avec la première branche inférieure (53,275,412,512).

2. Dispositif implantable (100,200,300,400,500,600,700,800), selon la revendication 1, **caractérisé en ce que** ledit au moins premier ressort latéral (20,212,312,450,540,640,820) a une hauteur H1 (H1',H1",H1‴) dans la position A et une hauteur H2 dans la position B, H2 étant différente de H1.

3. Dispositif implantable (100,200,300,400,500,600,700,800), selon l'une ou l'autre des revendications 1 et 2, **caractérisé en ce que** le premier ressort latéral (20,212,312,450,540,640,820) comprend au moins un matériau choisi parmi les matériaux à mémoire de forme.

4. Dispositif implantable (100,200,300,400,500,600,700,800), selon l'une quelconque des revendications 1 à 3, **caractérisé en ce que** le premier ressort latéral (20,212,312,450,540,640,800) comprend des premières portions supérieure (22) et inférieure (24) distantes de D1 (D1',D1",D1‴) dans la position A, et distantes de D2 dans la position B, D2 étant différente de D1 (D1',D1",D1‴), et **en ce que** le dispositif implantable (100,200,300,400,500,600,700), est positionné dans la position d'introduction par rapprochement ou écartement des premières portions supérieure (22) et inférieure (24) du premier ressort latéral (20,212,312,450,540,640,820).

5. Dispositif implantable (100,200,300,400,500,600,700,800), selon l'une quelconque des revendications 1 à 4, **caractérisé en ce que** le premier ressort latéral (20,212,312,450,540,640,820) s'étend d'un côté d'un plan de coupe sagittale (S) de la cale intervertébrale (10,230,330,430,530,630,730,810).

6. Dispositif implantable (100,200,300,400,500,600,700,800), selon l'une quelconque des revendications 1 à 5, **caractérisé en ce que** le ou les élément(s) longiligne(s) est/sont un ruban ou un fil.

7. Dispositif implantable (100,200,300,400,500,600,700,800), selon l'une quelconque des revendications 1 à 6, **caractérisé en ce que** la première branche intermédiaire (55,250,413,513) comprend des premières portions intermédiaires supérieure (56,514) et inférieure (57,515) convergeant vers une première zone intermédiaire (58,416,516) autour de laquelle lesdites premières portions intermédiaires supérieure (56,514) et inférieure (57,515) pivotent lors du passage de la position A vers la position B.

8. Dispositif implantable (100,200,300,400,500,700,800), selon l'une quelconque des revendications 1 à 7, **caractérisé en ce que** les premières portions intermédiaires supérieure (514) et inférieure (515) comprennent chacune une extrémité libre (517,518).

9. Dispositif implantable (100,200,300,400,500,700,800), selon la revendication 7 , **caractérisé en ce que** la première zone intermédiaire (58,416) est une zone de pliage (59) de la première branche intermédiaire (55,413).

10. Dispositif implantable selon l'une quelconque des revendications 1 à 9, **caractérisé en ce que** la première branche supérieure, la première branche inférieure, et la première branche intermédiaire, comprennent chacune au moins un élément longiligne au moins en partie métallique, éventuellement revêtu d'au moins un matériau polymère.

11. Dispositif implantable (100,200,300,400,500,600,700,800), selon l'une quelconque des revendications 1 à 10, **caractérisé en ce que** la première armature (50,255,410,510,640) comprend au moins une portion d'élément longiligne (65,313,315,470,480) faisant office de lame ressort, en particulier la première branche intermédiaire (55,250,413,513) comprend au moins une lame ressort.

12. Dispositif implantable selon l'une quelconque des revendications 1 à 9, **caractérisé en ce que** la première branche intermédiaire est une première branche intermédiaire polymérique exempte d'élément longiligne métallique, en particulier est en liaison avec les premières branches supérieure et inférieure de la première armature.

13. Dispositif implantable (100,200,300,400,500,600,700,800), selon l'une quelconque des revendications 1 à 12, **caractérisé en ce que** l'un au moins du ou desdits élément(s) longiligne(s) au moins en partie métallique (65,313,315,470,480) comprend au moins une portion vrillée ( 27,47) dans la position A ou B, et **en ce que** ladite portion vrillée (27,47) est dévrillée lors de son changement de position entre les positions A et B.

14. Dispositif implantable (100,200,300,400,700,800), selon la revendication 7, **caractérisé en ce que** la première branche intermédiaire (55,250,413) a une épaisseur e0 dans sa première zone intermédiaire (58,416) qui est inférieure à l'épaisseur e1 de la première portion intermédiaire supérieure (56) et/ou à l'épaisseur e2 de la première portion intermédiaire inférieure (57).

15. Dispositif implantable (100,200,300,400,500,600,700,800), selon l'une quelconque des revendications 1 à 14, caractérisé en ce quele second ressort latéral (40,214,314,460,560,620,840) comprend une seconde armature (60,265,420,520,660,842) comprenant un ou plusieurs élément(s) longiligne(s) au moins en partie métallique(s) (65,313,315,470,480).

16. Dispositif implantable (100,200,300,400,500,600,700,800), selon la revendication 15, **caractérisé en ce qu'**il comprend une armature principale (70,210,310) comprenant la première armature (50,255,410,510,640) et la seconde armature (60,265,420, 520,660,842), et **en ce que** l'armature principale (70,210,310) s'étend en partie à travers la cale intervertébrale (10,230,330,430, 530,630,730,810).

17. Dispositif implantable (100,200,300,400,500,600,700,800), selon l'une quelconque des revendications 1 à 16, **caractérisé en ce que** le premier ressort latéral (20,212,312,450,540,640) a une forme sensiblement de première boucle.

18. Dispositif implantable (400,500,600,700,800), selon l'une quelconque des revendications 1 à 17, **caractérisé en ce que** la première armature (410,510,640), et éventuellement la seconde armature (420,520,660,842), est/sont revêtue(s) en tout ou partie d'au moins un matériau choisi parmi : les polymères, les textiles et une combinaison de ces derniers.

19. Dispositif implantable (800) selon l'une quelconque des revendications 1 à 18, **caractérisé en ce que** le premier ressort latéral (820) comprend une partie crâniale (830) et une partie caudale (860), et **en ce que** la partie crâniale (830) est inclinée par rapport à la partie caudale (860), en particulier d'un angle α1 inférieur ou égal à 60°.

20. Dispositif implantable (800) selon la revendication19, **caractérisé en ce que** la partie crâniale (830) comprend une face antérieure comprenant une région passant par un plan Pa, et la partie caudale (860) comprend une face antérieure comprenant une région passant par un plan Pb, les plans Pa et Pb étant sécants.

21. Dispositif implantable (400,500,600,700,800), selon l'une quelconque des revendications 1 à 20, **caractérisé en ce que** la cale intervertébrale (10,230,330,430,530,630,730,810) comprend au moins un matériau polymère élastomérique, en particulier choisi parmi les silicones, ou les polyuréthanes.

22. Dispositif implantable, selon l'une quelconque des revendications 1 à 21, **caractérisé en ce que** la cale intervertébrale comprend une enveloppe extérieure textile, en particulier enveloppant au moins partiellement un noyau dans au moins un matériau polymère, en particulier élastomérique.

## Patentansprüche

1. Implantierbare Vorrichtung (100, 200, 300, 400, 500, 600, 700, 800), insbesondere zum Aufrechterhalten eines Zwischenwirbelabstands zwischen darüber und darunter liegenden Wirbeln (780, 790), umfassend:
- einen Zwischenwirbelkeil (10, 230, 330, 430, 530, 630, 730, 810), der eine obere Stützzone (12, 731, 812), insbesondere auf zumindest einem Abschnitt des Blattes des darüber liegenden Wirbels, und eine untere Stützzone (14, 732, 814), insbesondere auf zumindest einem Abschnitt des Blattes des darunter liegenden Wirbels, getrennt durch einen Mindestabstand Da zur Aufrechterhaltung eines Zwischenwirbelspalts umfasst, und
- eine erste seitliche Feder (20, 212, 312, 450, 540, 640, 820) und eine zweite seitliche Feder, die jeweils eine Position A in Bezug auf den Zwischenwirbelkeil (10, 230, 330, 430, 530, 630, 730, 810) in einer Einführungsposition der implantierbaren Vorrichtung (100, 200, 300, 400, 500, 600, 700, 800) und eine Position B in Bezug auf den Zwischenwirbelkeil (10, 230, 330, 430, 530, 630, 730, 810) in einer Implantationsposition der implantierbaren Vorrichtung aufweisen, wobei die Positionen A und B unterschiedlich sind, und dass die erste seitliche Feder (20, 212, 312, 450, 540, 640, 820) und die zweite seitliche Feder ausgestaltet sind zum:
- freien Verlaufen von der Position A zu der Position B und
- Bilden von Halteelementen, die in Bezug auf den Zwischenwirbelkeil (10, 230, 330, 430, 530, 630, 730, 810) angeordnet sind, um die Wanderung des Zwischenwirbelkeils (10, 230, 330, 430, 530, 630, 730, 810) zu dem Spinalkanal zumindest in der Position B zu blockieren,
**dadurch gekennzeichnet, dass**:
- die erste seitliche Feder (20, 212, 312, 450, 540, 640, 820) eine erste Armatur (50, 255, 410, 510, 640, 820) umfasst, die ein oder mehrere zumindest teilweise metallische längliche Elemente (65, 313, 315, 470, 480) umfasst, dass die erste Armatur (50, 255, 410, 510, 640, 820) einen ersten oberen Zweig (51, 270, 411, 511, 641) und einen ersten unteren Zweig (53, 275, 412, 512, 642) umfasst, die voneinander divergieren, und dass die erste Armatur (50, 255, 410, 510) einen ersten durchgehenden oder unterbrochenen Zwischenzweig (55, 250, 413, 513) in Verbindung mit dem ersten oberen Zweig (51, 270, 411, 511) und mit dem ersten unteren Zweig (53, 275, 412, 512) umfasst.

2. Implantierbare Vorrichtung (100, 200, 300, 400, 500, 600, 700, 800) nach Anspruch 1, **dadurch gekennzeichnet, dass** die zumindest erste seitliche Feder (20, 212, 312, 450, 540, 640, 820) eine Höhe H1 (H1', H1", H1‴) in der Position A und eine Höhe H2 in der Position B aufweist, wobei H2 verschieden von H1 ist.

3. Implantierbare Vorrichtung (100, 200, 300, 400, 500, 600, 700, 800) nach einem der Ansprüche 1 und 2, **dadurch gekennzeichnet, dass** die erste seitliche Feder (20, 212, 312, 450, 540, 640, 820) zumindest ein Material ausgewählt aus Formgedächtnismaterialien umfasst.

4. Implantierbare Vorrichtung (100, 200, 300, 400, 500, 600, 700, 800) nach einem der Ansprüche 1 bis 3, **dadurch gekennzeichnet, dass** die erste seitliche Feder (20, 212, 312, 450, 540, 640, 800) einen ersten oberen (22) und einen ersten unteren (24) Abschnitt entfernt von D1 (D1', D1", D1‴) in der Position A und entfernt von D2 in der Position B umfasst, wobei D2 verschieden von D1 (D1', D1", D1‴) ist, und dass die implantierbare Vorrichtung (100, 200, 300, 400, 500, 600, 700) in der Einführungsposition durch Aufeinanderzubewegen oder Auseinanderdrücken des ersten oberen (22) und des ersten unteren (24) Abschnittes der ersten seitlichen Feder (20, 212, 312, 450, 540, 640, 820) positioniert ist.

5. Implantierbare Vorrichtung (100, 200, 300, 400, 500, 600, 700, 800) nach einem der Ansprüche 1 bis 4, **dadurch gekennzeichnet, dass** sich die erste seitliche Feder (20, 212, 312, 450, 540, 640, 820) von einer Seite einer sagittalen Schnittebene (S) des Zwischenwirbelkeils (10, 230, 330, 430, 530, 630, 730, 810) erstreckt.

6. Implantierbare Vorrichtung (100, 200, 300, 400, 500, 600, 700, 800) nach einem der Ansprüche 1 bis 5, **dadurch gekennzeichnet, dass** das oder die längliche(n) Element(e) ein Band oder ein Faden ist/sind.

7. Implantierbare Vorrichtung (100, 200, 300, 400, 500, 600, 700, 800) nach einem der Ansprüche 1 bis 6, **dadurch gekennzeichnet, dass** der erste Zwischenzweig (55, 250, 413, 513) einen ersten oberen (56, 514) und einen ersten unteren (57, 515) Zwischenabschnitt umfasst, die zu einer ersten Zwischenzone (58, 416, 516) konvergieren, um welche der erste obere (56, 514) und der erste untere (57, 515) Zwischenabschnitt bei dem Verlaufen von der Position A zu der Position B schwenken.

8. Implantierbare Vorrichtung (100, 200, 300, 400, 500, 700, 800) nach einem der Ansprüche 1 bis 7, **dadurch gekennzeichnet, dass** der erste obere (514) und der erste untere (515) Zwischenabschnitt jeweils ein freies Ende (517, 518) umfassen.

9. Implantierbare Vorrichtung (100, 200, 300, 400, 500, 700, 800) nach Anspruch 7, **dadurch gekennzeichnet, dass** die erste Zwischenzone (58, 416) eine Faltungszone (59) des ersten Zwischenzweiges (55, 413) ist.

10. Implantierbare Vorrichtung nach einem der Ansprüche 1 bis 9, **dadurch gekennzeichnet, dass** der erste obere Zweig, der erste untere Zweig und der erste Zwischenzweig jeweils zumindest ein zumindest teilweise metallisches längliches Element umfassen optional mit zumindest einem Polymermaterial beschichtet.

11. Implantierbare Vorrichtung (100, 200, 300, 400, 500, 600, 700, 800) nach einem der Ansprüche 1 bis 10, **dadurch gekennzeichnet, dass** die erste Armatur (50, 255, 410, 510, 640) zumindest einen länglichen Elementabschnitt (65, 313, 315, 470, 480) umfasst, der als Blattfeder fungiert, insbesondere der erste Zwischenzweig (55, 250, 413, 513) zumindest eine Blattfeder umfasst.

12. Implantierbare Vorrichtung nach einem der Ansprüche 1 bis 9, **dadurch gekennzeichnet, dass** der erste Zwischenzweig ein erster Polymerzwischenzweig ohne metallisches längliches Element ist, insbesondere in Verbindung mit dem ersten oberen und dem ersten unteren Zweig der ersten Armatur ist.

13. Implantierbare Vorrichtung (100, 200, 300, 400, 500, 600, 700, 800) nach einem der Ansprüche 1 bis 12, **dadurch gekennzeichnet, dass** zumindest eines von dem oder den zumindest teilweise metallischen länglichen Element(en) (65, 313, 315, 470, 480) zumindest einen verdrehten Abschnitt (27, 47) in der Position A oder B umfasst und dass der verdrehte Abschnitt (27, 47) bei seinem Positionswechsel zwischen Position A und B ausgedreht wird.

14. Implantierbare Vorrichtung (100, 200, 300, 400, 700, 800) nach Anspruch 7, **dadurch gekennzeichnet, dass** der erste Zwischenzweig (55, 250, 413) eine Dicke e0 in seiner ersten Zwischenzone (58, 416) aufweist, die geringer als die Dicke e1 des ersten oberen Zwischenabschnittes (56) und/oder als die Dicke e2 des ersten unteren Zwischenabschnittes (57) ist.

15. Implantierbare Vorrichtung (100, 200, 300, 400, 500, 600, 700, 800) nach einem der Ansprüche 1 bis 14, **dadurch gekennzeichnet, dass** die zweite seitliche Feder (40, 214, 314, 460, 560, 620, 840) eine zweite Armatur (60, 265, 420, 520, 660, 842) umfasst, die ein oder mehrere zumindest teilweise metallische längliche Elemente (65, 313, 315, 470, 480) umfasst.

16. Implantierbare Vorrichtung (100, 200, 300, 400, 500, 600, 700, 800) nach Anspruch 15, **dadurch gekennzeichnet, dass** sie eine Hauptarmatur (70, 210, 310) umfasst, welche die erste Armatur (50, 255, 410, 510, 640) und die zweite Armatur (60, 265, 420, 520, 660, 842) umfasst, und dass sich die Hauptarmatur (70, 210, 310) teilweise quer über den Zwischenwirbelkeil (10, 230, 330, 430, 530, 630, 730, 810) erstreckt.

17. Implantierbare Vorrichtung (100, 200, 300, 400, 500, 600, 700, 800) nach einem der Ansprüche 1 bis 16, **dadurch gekennzeichnet, dass** die erste seitliche Feder (20, 212, 312, 450, 540, 640) eine Form im Wesentlichen einer ersten Schleife aufweist.

18. Implantierbare Vorrichtung (400, 500, 600, 700, 800) nach einem der Ansprüche 1 bis 17, **dadurch gekennzeichnet, dass** die erste Armatur (410, 510, 640) und optional die zweite Armatur (420, 520, 660, 842) vollständig oder teilweise mit zumindest einem Material beschichtet ist/sind, ausgewählt aus: Polymeren, Textilien und einer Kombination davon.

19. Implantierbare Vorrichtung (800) nach einem der Ansprüche 1 bis 18, **dadurch gekennzeichnet, dass** die erste seitliche Feder (820) einen kranialen Teil (830) und einen kaudalen Teil (860) umfasst und dass der kraniale Teil (830) in Bezug auf den kaudalen Teil (860) geneigt ist, insbesondere in einem Winkel α1 kleiner als oder gleich 60°.

20. Implantierbare Vorrichtung (800) nach Anspruch 19, **dadurch gekennzeichnet, dass** der kraniale Teil (830) eine anteriore Fläche umfasst, die eine Region umfasst, die durch eine Ebene Pa verläuft, und der kaudale Teil (860) eine anteriore Fläche umfasst, die eine Region umfasst, die durch eine Ebene Pb verläuft, wobei die Ebenen Pa und Pb einander schneiden.

21. Implantierbare Vorrichtung (400, 500, 600, 700, 800) nach einem der Ansprüche 1 bis 20, **dadurch gekennzeichnet, dass** der Zwischenwirbelkeil (10, 230, 330, 430, 530, 630, 730, 810) zumindest ein elastomeres Polymermaterial umfasst, insbesondere ausgewählt aus Silikonen oder Polyurethanen.

22. Implantierbare Vorrichtung nach einem der Ansprüche 1 bis 21, **dadurch gekennzeichnet, dass** der Zwischenwirbelkeil eine äußere Textilhülle umfasst, die insbesondere einen Kern in zumindest einem Polymermaterial, insbesondere elastomer, zumindest teilweise umhüllt.

## Claims

1. An implantable device (100, 200, 300, 400, 500, 600, 700, 800), especially, for maintaining an intervertebral space between overlying and underlying vertebrae (780, 790), comprising:
- an intervertebral wedge (10, 230, 330, 430, 530, 630, 730, 810) comprising an upper bearing zone (12, 731, 812), in particular over at least a portion of the lamina of the overlying vertebra, and a lower bearing zone (14, 732, 814), in particular over at least a portion of the lamina of the underlying vertebra, separated by a minimum distance Da to maintain an intervertebral space, and
- a first lateral spring (20, 212, 312, 450, 540, 640, 820), and a second lateral spring, each having a position A relative to the intervertebral wedge (10, 230, 330, 430, 530, 630, 730, 810) in a position for inserting the implantable device (100, 200, 300, 400, 500, 600, 700, 800), and a position B relative to the intervertebral wedge (10, 230, 330, 430, 530, 630, 730, 810), in a position for implanting the implantable device, the positions A and B being different, and in that the first lateral spring (20, 212, 312, 450, 540, 640, 820), and the second lateral spring, are configured:
- to freely move from position A to position B, and
- to form retention components arranged relative to the intervertebral wedge (10, 230, 330, 430, 530, 630, 730, 810) to block the migration of the intervertebral wedge (10, 230, 330, 430, 530, 630, 730, 810) toward the spinal canal at least in position B, **characterized in that**:
- the first lateral spring (20, 212, 312, 450, 540, 640, 820) comprises a first armature (50, 255, 410, 510, 640, 820) comprising one or more at least partially metallic elongated element(s) (65, 313, 315, 470, 480), **in that** the first armature (50, 255, 410, 510, 640,820) comprises a first upper arm (51, 270, 411, 511, 641) and a first lower arm (53, 275, 412, 512, 642) which diverge from each other, and **in that** the first armature (50, 255, 410, 510) comprises a first intermediate arm (55, 250, 413, 513), continuous or discontinuous, in connection with the first upper arm (51, 270, 411, 511) and with the first lower arm (53, 275, 412, 512).

2. The implantable device (100, 200, 300, 400, 500, 600, 700, 800), according to claim 1, **characterised in that** said at least first lateral spring (20, 212, 312, 450, 540, 640, 820) has a height H1 (H1',H1",H1‴) in position A and a height H2 in position B, H2 being different from H1.

3. The implantable device (100, 200, 300, 400, 500, 600, 700, 800), according to either one of claims 1 or 2, **characterised in that** the first lateral spring (20, 212, 312, 450, 540, 640, 820) comprises at least one material chosen from shape-memory materials.

4. The implantable device (100, 200, 300, 400, 500, 600, 700, 800) according to any one of claims 1 to 3, **characterised in that** the first lateral spring (20, 212, 312, 450, 540, 640, 800) comprises first upper (22) and lower (24) portions separated by D1 (D1', D1", D1‴) in position A, and separated by D2 in position B, D2 being different from D1 (D1', D1", D1‴), and **in that** the implantable device (100, 200, 300, 400, 500, 600, 700), is positioned in the insertion position by bringing together or separating the first upper (22) and lower (24) portions of the first lateral spring (20, 212, 312, 450, 540, 640, 820).

5. The implantable device (100, 200, 300, 400, 500, 600, 700, 800), according to any one of claims 1 to 4, **characterised in that** the first lateral spring (20, 212, 312, 450, 540, 640,820) extends from one side of a sagittal section plane (S) of the intervertebral wedge (10, 230, 330, 430, 530, 630, 730, 810).

6. The implantable device (100, 200, 300, 400, 500, 600, 700, 800), according to any one of claims 1 to 5, **characterised in that** the one or more elongated element(s) is/are a ribbon or a wire.

7. The implantable device (100, 200, 300, 400, 500, 600, 700, 800), according to any one of claims 1 to 6, **characterised in that** the first intermediate arm (55, 250, 413, 513) comprises first upper (56, 514) and lower (57, 515) intermediate portions converging towards a first intermediate zone (58, 416, 516) around which said first upper (56, 514) and lower (57, 515) intermediate portions pivot when moving from position A to position B.

8. The implantable device (100, 200, 300, 400, 500, 700, 800), according to any one of claims 1 to 7, **characterised in that** the first upper (514) and lower (515) intermediate portions each comprise a free end (517, 518).

9. The implantable device (100, 200, 300, 400, 500, 700, 800), according to claim 7, **characterised in that** the first intermediate zone (58, 416) is a bending zone (59) of the first intermediate arm (55, 413).

10. The implantable device (100, 200, 300, 400, 500, 600, 700, 800), according to any one of claims 1 to 9, **characterised in that** the first upper arm, the first lower arm, and the first intermediate arm, each comprise at least one at least partially metallic elongated element, optionally coated with at least one polymer material.

11. The implantable device (100, 200, 300, 400, 500, 600, 700, 800), according to any one of claims 1 to 10, **characterised in that** the first armature (50, 255, 410, 510, 640) comprises at least one portion of an elongated element (65, 313, 315, 470, 480) acting as a leaf spring, in particular the first intermediate arm (55, 250, 413, 513) comprises at least one leaf spring.

12. The implantable device (100, 200, 300, 400, 500, 600, 700, 800), according to any one of claims 1 to 9, **characterised in that** the first intermediate arm is a first polymeric intermediate arm free of metallic elongated element, in particular is connected with the first upper and lower arms of the first armature.

13. The implantable device (100, 200, 300, 400, 500, 600, 700, 800), according to any one of claims 1 to 12, **characterised in that** at least one of said at least partially metallic elongated element(s) (65, 313, 315, 470, 480) comprises at least one twisted portion (27, 47) in position A or B, and **in that** said twisted portion (27, 47) is untwisted when it changes position between positions A and B.

14. Implantable device (100, 200, 300, 400, 700, 800), according to claim 7, **characterised in that** the first intermediate arm (55, 250, 413) has a thickness e0 in its first intermediate zone (58, 416) which is less than the thickness e1 of the first upper intermediate portion (56) and/or than the thickness e2 of the first lower intermediate portion (57).

15. The implantable device (100, 200, 300, 400, 500, 600, 700, 800), according to any one of claims 1 to 14, **characterised in that** the second lateral spring (40, 214, 314, 460, 560, 620, 840) comprising a second armature (60, 265, 420, 520, 660, 842) comprising one or more at least partially metallic elongated element(s) (65, 313, 315, 470, 480).

16. The implantable device (100, 200, 300, 400, 500, 600, 700, 800), according to claim 15, **characterised in that** it comprises a main armature (70, 210, 310) comprising the first armature (50, 255, 410, 510, 640) and the second armature (60, 265, 420, 520, 660, 842), and **in that** the main armature (70, 210, 310) extends partly through the intervertebral wedge (10, 230, 330, 430, 530, 630, 730, 810).

17. The implantable device (100, 200, 300, 400, 500, 600, 700, 800), according to any one of claims 1 to 16, **characterised in that** the first lateral spring (20, 212, 312, 450, 540, 640) essentially has the shape of a first loop.

18. The implantable device (400, 500, 600, 700, 800), according to any one of claims 1 to 17, **characterised in that** the first armature (410, 510, 640), and optionally the second armature (420, 520, 660, 842), is/are coated completely or partially with at least one material chosen from: polymers, textiles and a combination thereof.

19. The implantable device (800) according to any one of claims 1 to 18, **characterised in that** the first lateral spring (820) comprises a cranial part (830) and a caudal part (860), and **in that** the cranial part (830) is inclined with respect to the caudal part (860), in particular by an angle α1 less than or equal to 60°.

20. The implantable device (800) according to claim19, **characterised in that** the cranial part (830) comprises an anterior face comprising a region passing through a plane Pa, and the caudal part (860) comprises an anterior face comprising a region passing through a plane Pb, the planes Pa and Pb being intersecting.

21. The implantable device (400, 500, 600, 700, 800) according to any one of claims 1 to 20, **characterised in that** the intervertebral wedge (10, 230, 330, 430, 530, 630, 730, 810) comprises at least one elastomeric polymer material, in particular chosen from silicones, or polyurethanes.

22. The implantable device, according to any one of claims 1 to 21, **characterised in that** the intervertebral wedge comprises an outer textile envelope, in particular at least partially enveloping a core in at least one polymer material, in particular elastomeric.
